(19) Europäisches Patentamt
European Patent Office
Office européen des brevets

(11) Veröffentlichungsnummer: **0 202 538**
**B1**

(12) # EUROPÄISCHE PATENTSCHRIFT

(45) Veröffentlichungstag der Patentschrift: **28.12.88**

(51) Int. Cl.[4]: **A 23 K 1/16**, C 07 D 333/38, C 07 D 333/68, C 07 D 333/78, C 07 D 333/80

(21) Anmeldenummer: **86106209.9**

(22) Anmeldetag: **06.05.86**

(54) **Leistungsfördernde Mittel.**

(30) Priorität: **17.05.85 DE 3517706**
**16.08.85 DE 3529247**

(43) Veröffentlichungstag der Anmeldung:
**26.11.86 Patentblatt 86/48**

(45) Bekanntmachung des Hinweises auf die Patenterteilung:
**28.12.88 Patentblatt 88/52**

(84) Benannte Vertragsstaaten:
**AT BE CH DE FR GB IT LI NL SE**

(56) Entgegenhaltungen:
**AT-B-311 994**
**DE-A-2 510 936**
**DE-A-2 645 613**
**DE-A-2 648 248**
**US-A-3 989 505**

**CHEMICAL ABSTRACTS, Band 91, Nr. 1, 2. Juli 1979, Seite 97, Nr. 814x, Columbus, Ohio, US**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Hallenbach, Werner, Dr., Kleiststrasse 10, D-4018 Langenfeld (DE)**
Erfinder: **Lindel, Hans, Dr., Carl-Duisberg-Strasse 321, D-5090 Leverkusen (DE)**
Erfinder: **Berschauer, Friedrich, Dr., Claudiusweg 9, D-5600 Wuppertal 1 (DE)**
Erfinder: **Scheer, Martin, Dr., Herberts-Katernberg 7, D-5600 Wuppertal 1 (DE)**
Erfinder: **de Jong, Anno, Dr., Stockmannsmühle 46, D-5600 Wuppertal 1 (DE)**

**EP 0 202 538 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft die Verwendung von teilweise bekannten Thienylharnstoffen und -isoharnstoffen als leistungsfördernde Mittel bei Tieren.

Thienylharnstoffe sind bereits bekannt geworden. Sie finden Verwendung als Herbizide und Pflanzenwachstumsregulatoren (vgl. DE-OS-2 040 579, 2 122 636, 2 627 935, 3 305 866, EP-OS-4931). Substituierte Tetrahydroiminobenzothien-4-yl-harnstoffe und ihre Verwendung zur Verbesserung der Futterausbeute und Erhöhung der Wachstumsgeschwindigkeit von Tieren sind bereits bekannt geworden. Sie befriedigen jedoch nicht in jedem Falle (DE-OS-2 645 613).

1. Es wurde gefunden, dass Thienylharnstoffe und -isoharnstoffe der Formel I

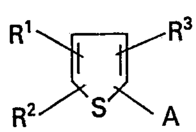

in welcher
A für die Reste Ia und Ib steht

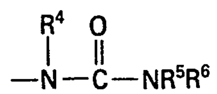 Ia

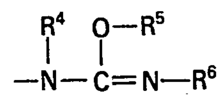 .Ib

$R^1$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Alkyl, Acyl, Aroyl, Aryl steht,

$R^2$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Acyl, Aroyl, Alkyl, Aryl steht,

$R^1$ und $R^2$ gemeinsam mit den angrenzenden C-Atomen für einen gegebenenfalls substituierten gesättigten oder ungesättigten carbocyclischen oder heterocyclischen Ring stehen, der gegebenenfalls eine Carbonylfunktion tragen kann,

$R^3$ für die Reste CN, $COOR^7$, $CONR^8R^9$, $COR^{10}$ steht,

$R^4$ für Wasserstoff oder Alkyl steht,

$R^5$ für Wasserstoff, gegebenenfalls substituiertes Alkyl, Cycloalkyl, Alkenyl, gegebenenfalls substituiertes Aryl oder Heteroaryl steht.

$R^6$ für Wasserstoff, gegebenenfalls substituiertes Alkyl, Cycloalkyl, Alkenyl, gegebenenfalls substituiertes Aryl oder Heteroaryl steht,

$R^7$ für Wasserstoff, gegebenenfalls substituiertes Alkyl, Cycloalkyl, Alkenyl, gegebenenfalls substituiertes Aryl steht,

$R^8$ für Wasserstoff oder Alkyl oder Cycloalkyl steht,

$R^9$ für Wasserstoff, gegebenenfalls substituiertes Alkyl, gegebenenfalls substituiertes Aryl steht,

$R^{10}$ für gegebenenfalls substituiertes Alkyl, gegebenenfalls substituiertes Aryl steht,
hervorragende leistungsfördernde Wirkung bei Tieren besitzen. Thienylharnstoffe und -isoharnstoffe der Formel I sind z. T. bekannt.

Thienylharnstoffe der Formel II

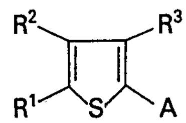 II

in welcher
A für den Rest Ia steht

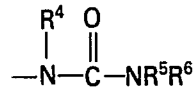 Ia

$R^1$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Acyl, Aroyl, Aryl steht,

$R^2$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Acyl, Aroyl, Alkyl, Aryl steht,

$R^1$ und $R^2$ zusammen mit den angrenzenden C-Atomen für einen gegebenenfalls substituierten gesättigten oder ungesättigten carbocyclischen Ring stehen, der gegebenenfalls eine Carbonylfunktion tragen kann,

$R^3$ für die Reste CN, $COOR^7$, $CONR^8R^9$, $COR^{10}$ steht,

$R^4$ für Wasserstoff oder Alkyl steht,

$R^5$ für Wasserstoff, gegebenenfalls substituiertes Alkyl, Cycloalkyl, Alkenyl, gegebenenfalls substituiertes Aryl steht,

$R^6$ für Wasserstoff, gegebenenfalls substituiertes Aklyl, gegebenenfalls substituiertes Aryl oder Heteroaryl steht,

$R^7$ für Wasserstoff, gegebenenfalls substituiertes Alkyl, Cycloalkyl, Alkenyl, gegebenenfalls substituiertes Aryl oder Heteroaryl steht,

$R^8$ für Wasserstoff, Alkyl oder Cycloalkyl steht,

$R^9$ für Wasserstoff, gegebenenfalls substituiertes Alkyl, Cycloalkyl, Alkenyl, gegebenenfalls substituiertes Aryl steht,

$R^{10}$ für gegebenenfalls substituiertes Alkyl, gegebenenfalls substituiertes Aryl steht,
können z.B. hergestellt werden, indem man Thienylisocyanate der Formel III

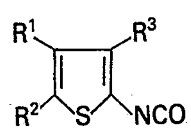 III

in welcher
$R^1$, $R^2$ und $R^3$ die oben angegebene Bedeutung haben, mit Aminen der Formel IV

H—$NR^5R^6$ , IV

in welcher
$R^5$ und $R^6$ die oben angegebene Bedeutung haben, umsetzt.

2. Es wurden die neuen Thienylisocyanate der Formel III gefunden

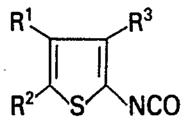

III

in welcher
$R^1$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Alkyl, Acyl, Aroyl, Aryl steht,
$R^2$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Alkyl, Acyl, Aroyl, Aryl steht,
$R^1$ und $R^2$ gemeinsam mit den angrenzenden C-Atomen für einen gegebenenfalls substituierten gesättigten oder ungesättigten carbocyclischen Ring stehen, der gegebenenfalls eine Carbonylfunktion tragen kann,
$R^3$ für die Reste $COOR^7$, $CONR^8R^9$, $COR^{10}$ steht,
$R^7$ für Wasserstoff, gegebenenfalls substituiertes Methyl, Cycloalkyl, $C_{2-4}$-Alkenyl, gegebenenfalls substituiertes Aryl steht,
$R^8$ für Wasserstoff, Alkyl oder Cycloalkyl steht,
$R^9$ für Wasserstoff, gegebenenfalls substituiertes Alkyl, gegebenenfalls substituiertes Aryl steht,
$R^{10}$ für gegebenenfalls substituiertes Alkyl, gegebenenfalls substituiertes Aryl steht,
mit Ausnahme von 3-Methoxycarbonyl-thien-2-yl-isocyanat.

3. Es wurde ferner gefunden, dass man die neuen Thienylisocyanate der Formel III gemäss 2 (oben) herstellen kann, indem man Thienylamine der Formel V

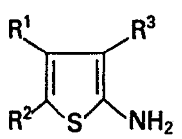

V

in welcher
$R^1$, $R^2$, $R^3$ die in 2 (oben) angegebene Bedeutung haben,
mit Phosgen umsetzt.

4. Es wurden ferner die neuen Thienylharnstoffe und -isoharnstoffe der Formel VI gefunden

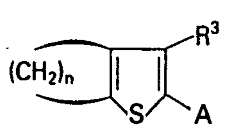

VI

in welcher
n für 3, 4, 5 oder 6 steht,
A für die Reste Ia und Ib steht

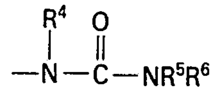

Ia

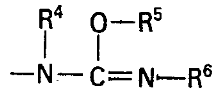

Ib

$R^3$ für den Fall, dass n für 3, 5, 6 steht, für die Reste CN, $COOR^7$, $CONR^8R^9$, $COR^{10}$ steht und für den Fall, dass n für 4 steht, für die Reste $COOCH_3$, $COO(C_{2-4}$-Alkenyl), $CONR^8R^9$, $COR^{10}$ steht,
$R^4$ für Wasserstoff oder Alkyl steht,
$R^5$ für Wasserstoff, gegebenenfalls substituiertes Alkyl, Cycloalkyl, Alkenyl, gegebenenfalls substituiertes Aryl oder Heteroaryl steht,
$R^6$ für Wasserstoff, gegebenenfalls substituiertes Alkyl, Cycloalkyl, Alkenyl, gegebenenfalls substituiertes Aryl oder Heteroaryl steht,
$R^7$ für Wasserstoff, gegebenenfalls substituiertes Alkyl, Cycloalkyl, Alkenyl, gegebenenfalls substituiertes Aryl steht,
$R^8$ für Wasserstoff, Alkyl oder Cycloalkyl steht,
$R^9$ für Wasserstoff, gegebenenfalls substituiertes Alkyl, gegebenenfalls substituiertes Aryl steht,
$R^{10}$ für gegebenenfalls substituiertes Alkyl, gegebenenfalls substituiertes Aryl steht.

5. Es wurde ferner gefunden, dass man die Thienylharnstoffe oder -isoharnstoffe der Formel VI erhält,

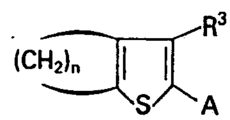

VI

in welcher
n für 3, 4, 5 oder 6 steht,
A für die Reste Ia und Ib steht

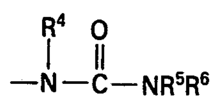

Ia

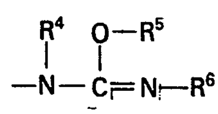

Ib

$R^3$ für den Fall, dass n für 3, 5, 6 steht, für die Reste CN, $COOR^7$, $CONR^8R^9$, $COR^{10}$ steht und für den Fall, dass n für 4 steht, für die Reste $COOCH_3$, $COO(C_{2-4}$-Alkenyl), $CONR^8R^9$, $COR^{10}$ steht,
$R^4$ für Wasserstoff oder Alkyl steht,
$R^5$ für Wasserstoff, gegebenenfalls substituiertes Alkyl, Cycloalkyl, Alkenyl, gegebenenfalls substituiertes Aryl oder Heteroaryl steht,
$R^6$ für Wasserstoff, gegebenenfalls substituiertes Alkyl, Cycloalkyl, Alkenyl, gegebenenfalls substituiertes Aryl oder Heteroaryl steht,
$R^7$ für Wasserstoff, gegebenenfalls substituiertes Alkyl, Cycloalkyl, Alkenyl, gegebenenfalls substituiertes Aryl steht,
$R^8$ für Wasserstoff, Alkyl oder Cycloalkyl steht,
$R^9$ für Wasserstoff, gegebenenfalls substitu-

iertes Alkyl, gegebenenfalls substituiertes Aryl steht,

$R^{10}$ für gegebenenfalls substituiertes Alkyl, gegebenenfalls substituiertes Aryl steht,

a) wenn man für den Fall, dass A für den Rest Ia steht und $R^5$ für Wasserstoff steht, Thienylamine der Formel VII

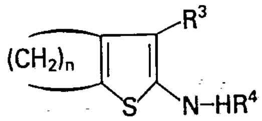 VII

in welcher

n, $R^3$ und $R^4$ die oben angegebene Bedeutung haben

mit Isocyanaten der Formel VIII

$OCN-R^6$ VIII

in welcher

$R^6$ die oben angegebene Bedeutung hat,

umsetzt, oder

b) wenn man für den Fall, dass A für den Rest Ia steht und $R^4$ für Wasserstoff steht, Thienylisocyanate der Formel IX

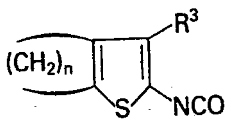 IX

in welcher

n und $R^3$ die oben angegebene Bedeutung haben,

mit Aminen der Formel IV

$H-NR^5R^6$ IV

in welcher

$R^5$ und $R^6$ die oben angegebene Bedeutung haben, umsetzt, oder

c) wenn man für den Fall, dass A für den Rest Ib steht, Thienylamine der Formel VII

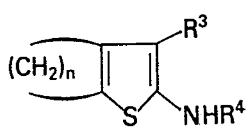 VII

in welcher

n, $R^3$ und $R^4$ die oben angegebene Bedeutung haben,

mit Imidokohlensäureesterhalogeniden der Formel X

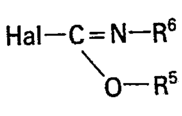 X

in welcher

$R^5$ und $R^6$ die oben angegebene Bedeutung haben, und

Hal für Halogen steht,

umsetzt.

Es war völlig überraschend, dass die Thienylharnstoffe der Formel I leistungsfördernde Eigenschaften bei Tieren aufweisen. Es gab aus dem Stand der Technik keinerlei Hinweis auf diese neue Verwendung der teilweise bekannten Thienylharnstoffe der Formel I.

Bevorzugt sind Thienylharnstoffe der Formel I, in welcher

A für die Reste Ia oder Ib steht,

$R^1$ für Wasserstoff, Halogen, Nitro, CN, $C_{1-4}$-Alkoxy, $C_{1-4}$-Alkylthio, gegebenfalls substituiertes $C_{1-6}$-Acyl, gegebenenfalls substituiertes Aroyl, insbesondere Benzoyl, für gegebenenfalls durch Halogen, $C_{1-4}$-Alkoxy, $C_{1-4}$-Alkylthio, Aryl, insbesondere Phenyl, Aryloxy, insbesondere Phenoxy, Arylthio, insbesondere Phenylthio, Amino, $C_{1-4}$-Alkylamino, Di-$C_{1-4}$-alkylamino, Arylamino, insbesondere Phenylamino substituiertes $C_{1-6}$-Alkyl sowie für Phenyl steht, wobei die Phenylreste gegebenenfalls einen oder mehrere der folgenden Substituenten tragen: Halogen, $C_{1-4}$-Alkyl, CN, $C_{1-4}$-Alkoxy, $C_{1-4}$-Alkylthio, Phenyl, Phenoxy, Phenylthio, Amino, $C_{1-4}$-Alkylamino, Di-$C_{1-4}$-alkylamino, $C_{1-4}$-Alkoxyalkyl, $C_{1-4}$-Halogenalkyl, $C_{1-4}$-Halogenalkoxy, $C_{1-4}$-Halogenalkylthio, Methylendioxy oder Ethylendioxy, die gegebenenfalls halogensubstituiert sind, Acyl.

$R^2$ für die bei $R^1$ aufgeführten Reste steht,

$R^1$ und $R^2$ gemeinsam mit den angrenzenden beiden C-Atomen für gesättigte oder ungesättigte carbocyclische Reste mit 5–8 Ringgliedern stehen, die gegebenenfalls durch OH, $C_{1-4}$-Alkyl, Halogen, Nitro, CN, $C_{1-4}$-Alkoxy, $C_{1-4}$-Alkylthio, Phenyl, Phenoxy, Phenylthio, Amino, $C_{1-4}$-Alkylamino, $C_{1-4}$-Dialkylamino, $C_{1-4}$-Halogenalkyl, $C_{1-4}$-Halogenalkoxy, $C_{1-4}$-Halogenalkylthio, $C_{1-4}$-Alkoxyalkyl substituiert sind und unter einer der Ringglieder, die nicht an den Thiophenring gebunden sind, eine Carbonylfunktion ($C=O$) tragen kann; für den Fall, dass $R^1$ und $R^2$ mit den angrenzenden C-Atomen einen heterocyclischen Ring bilden, hat dieser 5–6 Ringglieder und trägt O, S oder N als Heteroatome.

$R^3$ für die Reste CN, $COOR^7$, $CONR^8R^9$, $COR^{10}$ steht,

$R^4$ für Wasserstoff oder $C_{1-4}$-Alkyl steht,

$R^5$ für Wasserstoff, für gegebenenfalls durch Halogen, $C_{1-4}$-Alkoxy, $C_{1-4}$-Alkylthio, Aryl, insbesondere Phenyl, Aryloxy, insbesondere Phenoxy, Arylthio, insbesondere Phenylthio, Amino, $C_{1-4}$-Alkylamino, Di-$C_{1-4}$-alkylamino substituiertes $C_{1-6}$-Alkyl, $C_{3-8}$-Cycloalkyl, $C_{2-6}$-Alkenyl, ferner für Phenyl oder Naphthyl steht, wobei die Phenylreste gegebenenfalls einen oder mehrere der folgenden Substituenten tragen: Halogen, $C_{1-4}$-Alkyl, CN, $C_{1-4}$-Alkoxy, $C_{1-4}$-Alkylthio, Phenyl, Phenoxy, Phenylthio, Amino, $C_{1-4}$-Alkylamino, Di-$C_{1-4}$-alkylamino, $C_{1-4}$-Alkoxyalkyl, $C_{1-4}$-Halogenalkyl, $C_{1-4}$-Halogenalkoxy, $C_{1-4}$-Halogenalkylthio, Methylendioxy oder Ethylendioxy, die gegebenenfalls halogensubstituiert sind, sowie für Thienyl steht, das gegebenenfalls ein- oder mehrfach durch $C_{1-4}$-Alkyl, CN, Halogen, $C_{1-4}$-Alkoxycarbonyl substituiert ist,

$R^6$, $R^7$ und $R^9$ für die bei $R^5$ angeführten Reste stehen,
$R^8$ für Wasserstoff oder $C_{1-4}$-Alkyl, $C_{3-8}$-Cycloalkyl steht,
$R^{10}$ für die bei $R^5$ angeführten Reste, mit Ausnahme von Wasserstoff steht.

Besonders bevorzugt sind Verbindungen der Formel I, in welcher
A für die Reste Ia und Ib steht,
$R^1$ für Wasserstoff, $C_{1-6}$-Alkyl, das gegebenenfalls durch Fluor, Chlor oder Brom substituiert ist, Phenyl, das gegebenenfalls durch $C_{1-4}$-Alkyl, Halogen, $C_{1-4}$-Halogenalkyl, insbesondere Trifluormethyl, $C_{1-4}$-Halogenalkoxy, insbesondere Trifluormethoxy substituiert ist, für Nitro, Acyl, insbesondere Acetyl, steht,
$R^2$ für die bei $R^1$ angegebenen Reste steht,
$R^1$ und $R^2$ zusammen mit den angrenzenden C-Atomen für einen gesättigten 5–8-gliedrigen carbocyclischen Ring stehen, der gegebenenfalls durch $C_{1-4}$-Alkyl substituiert ist und gegebenenfalls an den Ringgliedern, die nicht an den Thiophenring gebunden sind, eine Carbonylfunktion trägt, sowie gemeinsam mit den angrenzenden C-Atomen für einen annellierten Benzolring stehen, der gegebenenfalls durch Halogen, insbesondere Chlor, Nitro, $C_{1-4}$-Alkyl substituiert ist,
$R^3$ für die Reste CN, $COOR^7$, $CONR^8R^9$, $COR^{10}$ steht,
$R^4$ und $R^6$ für Wasserstoff stehen,
$R^5$ für Wasserstoff, $C_{1-6}$-Alkyl, $C_{1-4}$-Alkylthio-$C_{1-4}$-alkyl, Cycloalkyl mit bis zu 8 C-Atopmen, $C_{2-4}$-Alkenyl, Phenyl, das gegebenenfalls durch $C_{1-4}$-Alkyl, $C_{1-4}$-Halogenalkyl, $C_{1-4}$-Alkoxy, Halogen, insbesondere Chlor, Nitro, substituiert ist, Naphthyl, Thienyl, das gegebenenfalls durch CN, $C_{1-4}$-Alkyl, $C_{1-4}$-Alkoxycarbonyl substituiert ist, steht,
$R^7$ für Wasserstoff, $C_{1-4}$-Alkyl, insbesondere Methyl, Ethyl, n-, t-Butyl, $C_{2-4}$-Alkenyl, insbesondere Allyl, sowie für Phenyl steht,
$R^8$ für Wasserstoff, $C_{1-4}$-Alkyl steht,
$R^9$ für Wasserstoff, $C_{1-4}$-Alkyl, insbesondere Methyl, Ethyl, steht,
$R^{10}$ für $C_{1-4}$-Alkyl, insbesondere Methyl, Phenyl steht.

Insbesondere seien Verbindungen der Formel I genannt, in welcher
A für den Rest der Formel Ia steht,
$R^1$ für Wasserstoff, $C_{1-5}$-Alkyl, insbesondere Methyl, Ethyl, Isopropyl, t-Butyl, n-Pentyl, Acetyl, Phenyl, Nitro steht,
$R^2$ für die bei $R^1$ angeführten Reste steht,
$R^1$ und $R^2$ gemeinsam für einen an den Thiophenring ankondensierten Cyclopentan-, Cyclohexan-, Cycloheptan-, Cyclooctan-, Cyclohexanon- oder Benzolring stehen, die gegebenenfalls durch $C_{1-4}$-Alkyl, insbesondere Methyl, Halogen, insbesondere Chlor, Nitro substituiert sein können, stehen,
$R^3$ für die Reste CN, $CONR^8R^9$, $COOR^7$, $COR^{10}$ steht,
$R^4$ und $R^6$ für Wasserstoff stehen,
$R^5$ für Wasserstoff, $C_{1-6}$-Alkyl, Cycloalkyl mit bis zu 6 C-Atomen, Phenyl, das gegebenenfalls durch Halogen, insbesondere Chlor, Nitro, Methyl, Methoxy, Trifluormethyl substituiert ist, steht,
$R^7$ für Wasserstoff, $C_{1-4}$-Alkyl, insbesondere Methyl, Ethyl, n-, t-Butyl, $C_{2-4}$-Alkenyl, insbesondere Allyl, sowie für Phenyl steht,
$R^8$ für Wasserstoff steht,
$R^9$ für Wasserstoff oder Methyl steht,
$R^{10}$ für Methyl oder Phenyl steht.

Im einzelnen seien neben den in den Beispielen genannten, die folgenden Verbindungen genannt:

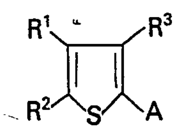

A = $-NH-CO-NHR^6$

| $R^1$ | $R^2$ | $R^3$ | $R^6$ |
|---|---|---|---|
| H | 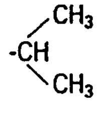 | 3-$CO_2Et$ | $-CH_3$ |
| H | 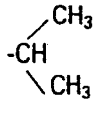 | 3-$CO_2Et$ | 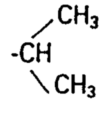 |
| H | 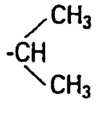 | 3-$CO_2Et$ | 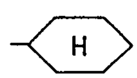 |

| $R^1$ | $R^2$ | $R^3$ | $R^6$ |
|---|---|---|---|
| H | 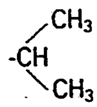 | 3-$CO_2$Et | 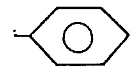 |
| H | 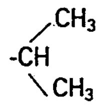 | 3-$CO_2$Et | sec-Butyl |
| H | 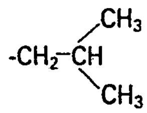 | 3-$CO_2$Et | -$CH_3$ |
| H | 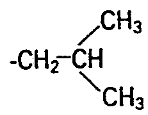 | 3-$CO_2$Et | 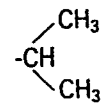 |
| H | 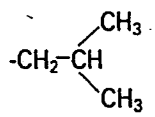 | 3-$CO_2$Et | 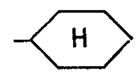 |
| H | 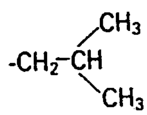 | 3-$CO_2$Et | 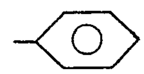 |
| H | 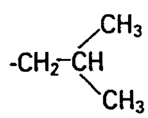 | 3-$CO_2$Et | sec-Butyl |
| H | 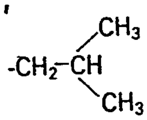 | 3-$CO_2$Et | tert.-Butyl |
| H | 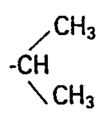 | 3-$CO_2$Et | tert.-Butyl |
| -$CH_3$ | -Et | 3-$CO_2$Et | 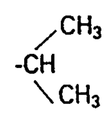 |
| -$CH_3$ | -Et | 3-$CO_2$Et | 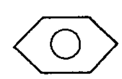 |
| $-(-CH_2-)-_3$ | | $CONH_2$ | $CH_3$ |
| $-(-CH_2-)-_3$ | | $CONH_2$ | 1-Propyl |
| $-(-CH_2-)-_3$ | | $CONH_2$ | n-Butyl |
| $-(-CH_2-)-_3$ | | $CONH_2$ | Cyclohexyl |
| $-(-CH_2-)-_3$ | | $CONH_2$ | Phenyl |
| $-(-CH_2-)-_3$ | | $CONH_2$ | 4-Chlorphenyl |
| $-(-CH_2-)-_4$ | | $CONHC_2H_5$ | $CH_3$ |
| $-(-CH_2-)-_4$ | | $CONHC_2H_5$ | 1-Propyl |
| $-(-CH_2-S-CH_2CH_2)-$ | | $CONH_2$ | $CH_3$ |
| $-(-CH_2-O-CH_2CH_2)-$ | | $CONH_2$ | $CH_3$ |
| $-(-CH_2-NH-CH_2CH_2)-$ | | $COOC_2H_5$ | $CH_3$ |

$A = -NH-CO-NR^5R^6$

| $R^1$ | $R^2$ | $R^3$ | $R^5$ | $R^6$ |
|---|---|---|---|---|
| $-(-CH_2-)-_4$ | | $COOCH_3$ | $CH_3$ | $CH_3$ |
| $-(-CH_2-)-_4$ | | $COOCH_3$ | $CH_3$ | $C_2H_5$ |
| $-(-CH_2-)-_4$ | | $COOCH_3$ | $C_2H_5$ | $C_2H_5$ |
| $-(-CH_2-)-_4$ | | $CONH_2$ | $CH_3$ | $CH_3$ |
| $-(-CH_2-)-_4$ | | $CONH_2$ | $CH_3$ | $C_2H_5$ |
| $-(-CH_2-)-_4$ | | $CONH_2$ | $C_2H_5$ | $C_2H_5$ |
| $-(-CH_2-)-_4$ | | CN | $CH_3$ | $CH_3$ |
| $-(-CH_2-)-_4$ | | CN | $CH_3$ | $C_2H_5$ |
| $-(-CH_2-)-_4$ | | CN | $C_2H_5$ | $C_2H_5$ |
| $-(-CH_2-)-_5$ | | $COOCH_3$ | $CH_3$ | $CH_3$ |
| $-(-CH_2-)-_5$ | | $COOCH_3$ | $H_3$ | $C_2H_5$ |
| $-(-CH_2-)-_5$ | | $COOCH_3$ | $C_2H_5$ | $C_2H_5$ |
| $-(-CH_2-)-_5$ | | $CONH_2$ | $CH_3$ | $CH_3$ |
| $-(-CH_2-)-_5$ | | $CONH_2$ | $CH_3$ | $C_2H_5$ |
| $-(-CH_2-)-_5$ | | $CONH_2$ | $C_2H_5$ | $C_2H_5$ |
| $-(-CH_2-)-_5$ | | CN | $CH_3$ | $CH_3$ |
| $-(-CH_2-)-_5$ | | CN | $C_2H_5$ | $C_2H_5$ |

| $R^1$ | $R^2$ | $R^3$ | R6 ($R^5$ = H) |
|---|---|---|---|
| H | $-CH_3$ | $3-CO_2Et$ | 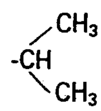 |
| H | $-CH_3$ | $3-CO_2Et$ | $-CH_3$ |
| H | $-CH_3$ | $3-CO_2Et$ | 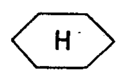 |
| H | $-CH_3$ | $3-CO_2Et$ | 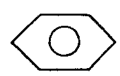 |

| $R^1$ | $R^2$ | $R^3$ | $R^6$ |
|---|---|---|---|
| 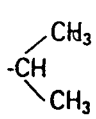 | H | 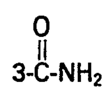 | $-CH_3$ |
| 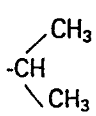 | H | 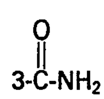 | 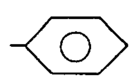 |
| 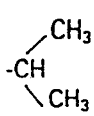 | H | 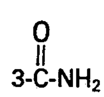 | 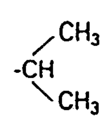 |
| H | -Et | $3-CO_2Et$ | $-CH_3$ |

| $R^1$ | $R^2$ | $R^3$ | $R^6$ |
|---|---|---|---|
| H | -Et | 3-$CO_2$Et | 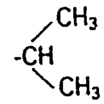 |
| H | -Et | 3-$CO_2$Et | 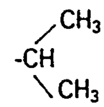 |
| H | -Et | 3-$CO_2$Et | 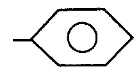 |
| H | -Et | 3-$CO_2$Et | tert.-Butyl |
| | | | ($R^5$) ($R^6$) |
| H | -Et | 3-$CO_2$Et | -$CH_3$, -$CH_3$ |
| -Et | -$CH_3$ | 3-$CO_2$Et | -$CH_3$, -$CH_3$ |
| $CH_3$ | H | $COOC_2H_5$ | $CH_3$ |
| $CH_3$ | H | $COOC_2H_5$ | i-Propyl |
| $CH_3$ | H | $COOC_2H_5$ | i-Butyl |
| $CH_3$ | H | $COOC_2H_5$ | Cyclopentyl |
| $CH_3$ | H | $COOC_2H_5$ | Cyclohexyl |
| $CH_3$ | H | $COOC_2H_5$ | Phenyl |
| $CH_3$ | H | $COOC_2H_5$ | 4-Methoxyphenyl |
| H | n-$C_5H_{11}$ | $COOC_2H_5$ | $CH_3$ |
| H | n-$C_5H_{11}$ | $COOC_2H_5$ | i-Propyl |
| H | n-$C_5H_{11}$ | $COOC_2H_5$ | i-Butyl |
| H | n-$C_5H_{11}$ | $COOC_2H_5$ | Cyclopentyl |
| H | n-$C_5H_{11}$ | $COOC_2H_5$ | Cyclohexyl |
| H | n-$C_5H_{11}$ | $COOC_2H_5$ | Phenyl |
| H | n-$C_5H_{11}$ | $COOC_2H_5$ | 4-Chlorphenyl |
| H | n-$C_5H_{11}$ | $COOC_2H_5$ | 4-Methoxyphenyl |
| H | Phenyl | 3-$COOC_2H_5$ | Cyclopropyl |

$$A = -NH-\overset{\overset{O-R^5}{|}}{C}=NR^6$$

| $R^1$ | $R^2$ | $R^3$ | $R^5$ | $R^6$ |
|---|---|---|---|---|
| -$CH_3$ | -$CH_3$ | 3-$CO_2$Et | -Et | -$CH_3$ |
| -H | 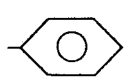 | 3-$CO_2$Et | -Et | -$CH_3$ |
| -H | -H | 3-$CO_2$Et | -Me | 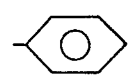 |

Die Thienylharnstoffe der Formel I sind teilweise bekannt. Sie lassen sich analog zu bekannten Verfahren herstellen (DE-OS-2 122 636, 2 627 935).

Die Thienylverbindungen der Formel II, in welcher der Rest A für den Harnstoffrest der Formel Ia in 2-Stellung des Thienylrings steht, lassen sich besonders vorteilhaft herstellen, indem man Thienyl-2-isocyanat der Formel III mit den Aminen der Formel IV umsetzt (vgl. Verfahren 2 oben).

Verwendet man 2-Isocyanato-3-cyano-4,5-tetramethylen-thiophen und Methylamin, lässt sich der Reaktionsverlauf durch folgendes Reaktionsschema darstellen:

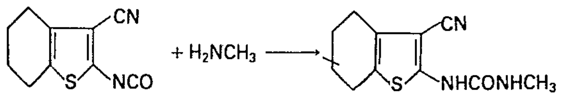

Als Verbindungen der Formel III werden bevorzugt diejenigen eingesetzt, die in den Substituenten $R^1$, $R^2$ und $R^3$ die bei den Verbindungen der Formel I genannten bevorzugten Bedeutungen besitzen. Die Verbindungen der Formel III sind neu. Ihre Herstellung erfolgt nach dem unter 4 angegebenen Verfahren, das weiter unten näher erläutert wird.

Im einzelnen seien neben den in den Beispielen genannten die folgenden Verbindungen der Formel III genannt:

2-Isocyanato-3-cyano-thiophen
2-Isocyanato-3-carbethoxy-5-isobutyl-thiophen
2-Isocyanato-3-cyano-4,5-trimethylen-thiophen
2-Isocyanato-3-methoxycarbonyl-4,5-trimethylen-thiophen
2-Isocyanato-3-ethoxycarbonyl-4,5-trimethylen-thiophen
2-Isocyanato-3-t-butoxycarbonyl-4,5-trimethylen-thiophen
2-Isocyanato-3-cyano-4,5-pentamethylen-thiophen
2-Isocyanato-3-methoxycarbonyl-4,5-pentamethylen-thiophen
2-Isocyanato-3-ethoxycarbonyl-4,5-pentamethylen-thiophen
2-Isocyanato-3-t-butoxycarbonyl-4,5-pentamethylen-thiophen
2-Isocyanato-3-carbethoxy-5-phenyl-thiophen
2-Isocyanato-3-carbethoxy-4-methyl-5-phenyl-thiophen.

Als Verbindungen der Formel IV werden bevorzugt diejenigen eingesetzt, die in den Substituenten $R^5$ und $R^6$ die bei den Verbindungen der Formel I genannten bevorzugten Bedeutungen haben. Die Verbindungen der Formel IV sind bekannte Verbindungen der organischen Chemie.

Im einzelnen seien folgende Verbindungen der Formel IV genannt:

Ammoniak, Methylamin, Dimethylamin, Ethylamin, Diethylamin, n-Propylamin, Di-n-propylamin, Isopropylamin, Di-isopropylamin, n-Butylamin, i-Butylamin, sec-Butylamin, t-Butylamin, Cyclopentylamin, Cyclohexylamin, Anilin, 2-Chloranilin, 3-Chloranilin, 4-Chloranilin, 2-Nitroanilin, 3-Nitroanilin, 4-Nitroanilin, 2-Methylanilin, 3-Methylanilin, 4-Methylanilin, 2-Methoxyanilin, 3-Methoxyanilin, 4-Methoxyanilin, 2-Trifluormethylanilin, 3-Trifluormethylanilin, 4-Trifluormethylanilin.

Zur Herstellung der Thienylharnstoffe der Formel II werden die Thienylisocyanate der Formel III und die Amine der Formel IV in etwa äquimolaren Mengen umgesetzt. Ein Überschuss der einen oder der anderen Komponente bringt keine wesentlichen Vorteile.

Die Umsetzung kann mit oder ohne Verdünnungsmittel erfolgen. Als Verdünnungsmittel seien genannt:

Alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, ausserdem Ester, wie Essigsäure-methylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Dimethylsulfoxid, Tetramethylensulfon und Hexamethylphosphorsäuretriamid.

Zur Beschleunigung des Reaktionsverlaufs können Katalysatoren zugesetzt werden. Als solche sind geeignet: z. B. tertiäre Amine wie Pyridin, 4-Dimethylaminopyridin, Triethylamin, Triethylendiamin, Trimethylen-tetrahydropyridimidin; ferner Zinn-II- und Zinn-IV-Verbindungen wie Zinn-II-octoat oder Zinn-IV-chlorid. Die als Reaktionsbeschleuniger genannten tertiären Amine, z. B. Pyridin, können auch als Lösungsmittel verwendet werden.

Die Reaktionstemperaturen können in einem grösseren Temperaturbereich variiert werden. Im allgemeinen arbeitet man zwischen 0 und 120 °C, vorzugsweise zwischen 20 und 70 °C.

Normalerweise arbeitet man unter Normaldruck, jedoch kann es zweckmässig sein, z. B. beim Einsatz niedrig siedender Amine, in geschlossenen Gefässen unter Druck zu arbeiten.

Bei der Durchführung des erfindungsgemässen Verfahrens setzt man die Ausgangsstoffe im allgemeinen in stöchiometrischen Verhältnissen ein, günstig ist jedoch ein geringer Überschuss des Amins. Die Katalysatoren werden vorzugsweise in Mengen von 0,01 bis 0,1 Mol pro Mol der Reaktionskomponenten angewandt, jedoch sind auch grössere Mengen, z. B. der tertiären Amine, anwendbar.

Die Reaktionsprodukte werden isoliert, indem man aus den entsprechenden Lösungsmitteln direkt ausfallende Produkte filtriert oder indem man das Lösungsmittel abdestilliert.

Wie bereits erwähnt, sind die Thienylisocyanate der Formel III neu. Bevorzugt sind Thienylisocyanate der Formel III, die in den Substituenten $R^1$–$R^3$, die bei den Verbindungen der Formel I für die Substituenten $R^1$–$R^3$ angegebenen bevorzugten Bedeutungen haben. Bevorzugte Verbindungen der Formel III sind im einzelnen die im Verfahren 2 angegebenen Verbindungen.

Thienylisocyanate der Formel III lassen sich durch Umsetzung der entsprechenden Thienylamine der Formel V mit Phosgen herstellen. Verwendet man 2-Amino-3-acetyl-4,5-tetramethylenthiophen und Phosgen, lässt sich der Reaktionsablauf durch folgendes Reaktionsschema darstellen:

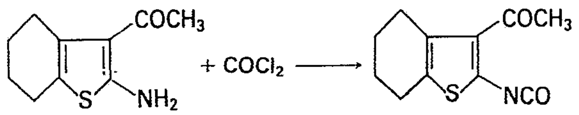

Als Thienylamine der Formel V werden bevorzugt diejenigen eingesetzt, die in den Substituenten $R^1$–$R^3$ die bei den Verbindungen der Formel I angegebenen bevorzugten Bedeutungen haben. Die Verbindungen der Formel V sind bekannt oder lassen sich analog zu bekannten Verfahren herstellen [K. Gewald et al. Chem. Ber. 98 (1965), S. 3571, Chem. Ber. 99 (1966), S. 94, EP-OS-4931].

Im einzelnen seien folgende Verbindungen der Formel V genannt:

2-Amino-3-cyano-4,5-trimethylen-thiophen
2-Amino-3-methoxycarbonyl-4,5-trimethylen-thiophen
2-Amino-3-ethoxycarbonyl-4,5-trimethylen-thiophen
2-Amino-3-t-butoxy-carbonyl-4,5-trimethylen-thiophen
2-Amino-3-cyano-4,5-tetramethylen-thiophen
2-Amino-3-methoxycarbonyl-4,5-tetramethylen-thiophen
2-Amino-3-ethoxycarbonyl-4,5-tetramethylen-thiophen
2-Amino-3-t-butoxycarbonyl-4,5-tetramethylen-thiophen
2-Amino-3-methoxycarbonyl-4,5-pentamethylen-thiophen
2-Amino-3-ethoxycarbonyl-4,5-pentamethylen-thiophen
2-Amino-3-t-butoxycarbonyl-4,5-pentamethylen-thiophen
2-Amino-3-carbethoxy-4-methyl-5-phenyl-thiophen
2-Amino-3-carbethoxy-4-methyl-5-ethyl-thiophen
2-Amino-3-carbethoxy-5-n-butyl-thiophen
2-Amino-3-carbethoxy-5-isobutyl-thiophen
2-Amino-3-carbethoxy-4-ethyl-5-methyl-thiophen
2-Amino-3-carbethoxy-5-phenyl-thiophen
2-Amino-3-carbethoxy-5-ethylthiophen
2-Amino-3-carbethoxy-5-isopropylthiophen

Die Umsetzung der Amine der Formel V mit Phosgen kann mit oder ohne Verdünnungsmittel erfolgen.

Als Verdünnungsmittel seien genannt: inerte organische Lösungsmittel, insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol, o-Dichlorbenzol.

Die Umsetzung erfolgt bei –20 °C bis +180 °C, bevorzugt bei –10 °C bis +100 °C. Es kann bei Normaldruck oder bei erhöhtem Druck gearbeitet werden.

Die Ausgangsstoffe werden in äquimolaren Mengen eingesetzt, bevorzugt ist ein Überschuss an Phosgen von 2–3 Mol pro Mol Amin der Formel V.

Die Reaktion wird ohne oder in Gegenwart von Säurebindemitteln durchgeführt. Säurebindemittel sind bevorzugt z. B. tertiäre Amine wie Pyridin, Dimethylanilin.

Die Amine der Formel V werden zu einer Lösung von Phosgen zugegeben und gegebenenfalls unter weiterem Einleiten von Phosgen umgesetzt. Die Umsetzung kann auch ohne Lösungsmittel durchgeführt werden.

Wie bereits erwähnt, sind die Thienylharnstoffe der Formel VI neu.

Bevorzugt sind Thienylharnstoffe der Formel VI, in der die Reste $R^3$ und A die bei den Verbindungen der Formel I angegebenen bevorzugten Bedeutungen haben. Im einzelnen seien die weiter vorne aufgeführten Thienylharnstoffe genannt.

Thienylharnstoffe der Formel VI, in welcher A für den Rest Ia steht und $R^4$ für Wasserstoff steht, lassen sich nach dem weiter oben beschriebenen Verfahren aus den entsprechenden Thienylisocyanaten und den entsprechenden Aminen herstellen. Einzelheiten dieses Verfahrens sind bereits weiter oben angegeben.

Thienylharnstoffe der Formel VI, in welcher A für den Rest Ia steht und $R^5$ für Wasserstoff steht, lassen sich aus den entsprechenden Thienylaminen der Formel VII durch Umsetzung mit Isocyanaten der Formel VIII herstellen. Verwendet man 2-Methylamino-3-methoxycarbonyl-4,5-trimethylenthiophen und Phenylisocyanat, lässt sich der Reaktionsablauf durch das folgende Reaktionsschema wiedergeben:

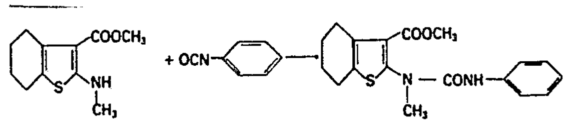

Die als Ausgangsprodukte zu verwendenden Thienylamine der Formel VII sind bekannt oder lassen sich analog zu bekannten Verfahren herstellen [K. Gewald Chem. Ber. 98 (1965), S. 3571, Chem. Ber. 99 (1966), S. 94, EP-OS-4931, G. Coppola et al. J. Heterocycl. Chem. 1982, S. 717).

Es werden bevorzugt die Thienylamine der Formel VII eingesetzt, die in den Substituenten $R^3$ und $R^4$ die bei den Verbindungen der Formel I angegebenen bevorzugten Bedeutungen haben.

Im einzelnen seien die auf dieser Seite aufgeführten Verbindungen der Formel V genannt.

Die als Ausgangsprodukte zu verwendenden Isocyanate sind bekannt. Als Beispiele seien im einzelnen genannt: Methylisocyanat, Ethyl-, n-Propyl-, Isopropyl-, n-Butyl-, Isobutyl-, tert.-Butyl- und Phenylisocyanat, 3-Chlorphenylisocyanat, 4-Chlorphenylisocyanat, 2,6-Dichlorphenylisocyanat.

Die erfindungsgemässe Umsetzung zwischen den Thienylaminen und den Isocyanaten führt man vorzugsweise in Gegenwart eines Verdünnungsmittels durch. Als solche eignen sich alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, ausserdem Ester, wie Essigsäure-methylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Dimethylsulfoxid, Tetramethylensulfon und Hexamethylphosphorsäuretriamid.

Zur Beschleunigung des Reaktionsverlaufs können Katalysatoren zugesetzt werden. Als solche sind geeignet: z. B. tertiäre Amine wie Pyridin, 4-Dimethylaminopyridin, Triethylamin, Triethylendiamin, Trimethylen-tetrahydropyrimidin; ferner Zinn-II- und Zinn-IV-Verbindungen wie Zinn-II-octoat oder Zinn-IV-chlorid. Die als Reaktionsbeschleuniger genannten tertiären Amine, z. B. Pyridin, können auch als Lösungsmittel verwendet werden.

Die Reaktionstemperaturen können in einem grösseren Temperaturbereich variiert werden. Im allgemeinen arbeitet man zwischen 0 und 120 °C, vorzugsweise zwischen 20 und 70 °C.

Normalerweise arbeitet man unter Normaldruck, jedoch kann es zweckmässig sein, z. B. beim Einsatz niedrig siedender Isocyanate, in geschlossenen Gefässen unter Druck zu arbeiten.

Bei der Durchführung des erfindungsgemässen Verfahrens setzt man die Ausgangsstoffe im allgemeinen in stöchiometrischen Verhältnissen ein, günstig ist jedoch ein geringer Überschuss des Isocyanats. Die Katalysatoren werden vorzugsweise in Mengen von 0,01 bis 0,1 Mol pro Mol der Reaktionskomponenten angewandt, jedoch sind auch grössere Mengen, z. B. der tertiären Amine, anwendbar.

Die Reaktionsprodukte werden isoliert, indem man aus den entsprechenden Lösungsmitteln direkt ausfallende Produkte filtriert oder indem man das Lösungsmittel abdestilliert.

Thienylisoharnstoffe der Formel VI, in welcher A für den Rest Ib steht, lassen sich aus den entsprechenden Thienylaminen der Formel VII durch Umsetzung mit den entsprechenden Imidokohlensäureesterhalogeniden der Formel X herstellen. Verwendet man 2-Ethylamino-3-benzoyl-4,5-hexamethylenthiophen und N-Phenyl-imidokohlensäureethylesterchlorid, lässt sich der Reaktionsablauf durch das folgende Reaktionsschema wiedergeben:

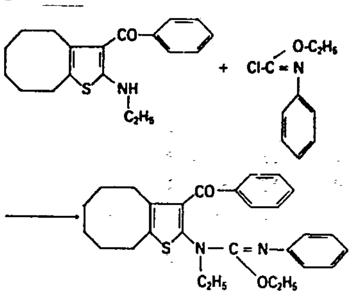

Es werden bevorzugt die weiter oben als bevorzugt angegebenen Thienylamine eingesetzt.

Imidokohlensäureesterhalogenide sind bekannt.

In Formel X haben $R^5$ und $R^6$ bevorzugt die weiter oben angegebenen bevorzugten Bedeutungen.

Halogen steht insbesondere für Chlor.

Im einzelnen seien folgende Imidokohlensäureesterhalogenide genannt: N-Methylimidokohlensäureethylesterchlorid, N-Ethyl-imidokohlensäureethylesterchlorid, N-Propyl-imidokohlensäureestermethylesterchlorid, N-Phenylimidokohlensäureethylesterchlorid.

Die Umsetzung erfolgt gegebenenfalls in Gegenwart von Säureakzeptoren, Katalysatoren und Verdünnungsmitteln.

Die Verbindungen der Formel VII und X werden bevorzugt äquimolar eingesetzt. Ein Überschuss der einen oder anderen Komponente bringt keinen wesentlichen Vorteil.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobhutylketon, ausserdem Ester, wie Essigsäure-methylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Dimethylsulfoxid, Tetramethylensulfon und Hexamethylphosphorsäuretriamid.

Als Säureakzeptoren können alle üblichen Säurebindemittel verwendet werden. Hierzu gehören vorzugsweise Alkalicarbonate, -hydroxide oder -alkoholate, wie Natrium- oder Kaliumcarbonat, Natrium- und Kaliumhydroxid, Natrium- und Kaliummethylat bzw. -ethylat, ferner aliphatische, aromatische oder heterocyclische Amine, beispielsweise Trimethylamin, Triethylamin, Tributylamin, Dimethylanilin, Dimethylbenzylamin, Pyridin und 4-Dimethylaminopyridin.

Als Katalysatoren können Verbindungen verwendet werden, welche gewöhnlich bei Reaktionen in Zweiphasensystemen aus Wasser und mit Wasser nicht mischbaren organischen Lösungsmitteln zum Phasentransfer von Reaktanden dienen (Phasentransferkatalysatoren). Als solche sind vor allem Tetraalkyl- und Trialkylaralkyl-ammoniumsalze mit vorzugsweise 1 bis 10, insbesondere 1 bis 8 Kohlenstoffen je Alkylgruppe, vorzugsweise Phenyl als Arylbestandteil der Aralkylgruppe und vorzugsweise 1 bis 4, insbesondere 1 oder 2 Kohlenstoffatomen im Alkylteil der Aralkylgruppen bevorzugt. Hierbei kommen vor allem die Halogenide, wie Chloride, Bromide und Iodide,

vorzugsweise die Chloride und Bromide in Frage. Beispielhaft seien Tetrabutylammoniumbromid, Benzyl-triethylammoniumchlorid und Methyltrioctylammoniumchlorid genannt.

Die Reaktionstemperatur wird zwischen etwa 0 und 130 °C, vorzugsweise zwischen etwa 20 und 60 °C gehalten. Das Verfahren wird vorzugsweise bei Normaldruck durchgeführt. Die Aufarbeitung erfolgt in üblicher Weise.

Die Wirkstoffe werden als Leistungsförderer bei Tieren zur Förderung und Beschleunigung des Wachstums, der Milch- und Wollproduktion sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier- und Hobbytieren verwendet.

Zu den Nutz- und Zuchttieren zählen Säugetiere wie z. B. Rinder, Schweine, Pferde, Schafe, Ziegen, Kaninchen, Hasen, Damwild, Pelztiere wie Nerze, Chinchilla, Geflügel wie z. B. Hühner, Puten, Gänse, Enten, Tauben, Fische wie z. B. Karpfen, Forellen, Lachse, Aale, Schleien, Hechte, Reptilien wie z. B. Schlangen und Krokodile.

Zu den Zier- und Hobbytieren zählen Säugetiere wie Hunde und Katzen, Vögel wie Papageien, Kanarienvögel, Fische wie Zier- und Aquarienfische, z. B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. bei kontinuierlicher Verabreichung kann die Anwendung ein- oder mehrmals täglich in regelmässigen oder unregelmässigen Abständen erfolgen.

Die Verabreichung erfolgt oral oder parenteral in dafür geeigneten Formulierungen oder in reiner Form. Orale Formulierungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm – 100%, bevorzugt von 0,01 ppm – 1%.

Parenterale Formulierungen sind Injektionen in Form von Lösungen, Emulsionen und Suspensionen sowie Implantate.

Die Wirkstoffe können in den Formulierungen allein oder in Mischung mit anderen Wirkstoffen, Mineralsalzen, Spurenelementen, Vitaminen, Eiweissstoffen, Farbstoffen, Fetten oder Geschmackstoffen vorliegen.

Die Konzentration der Wirkstoffe im Futter beträgt normalerweise etwa 0,01–500 ppm, bevorzugt 0,1–50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemässen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_2 \times H_2O$, 140 mg Zn $SO_4 \times 7\,H_2O$, 100 mg Fe $SO_4 \times 7\,H_2O$ und 200 mg Cu $SO_4 \times 5\,H_2O$.

2,5 g Wirkstoff-Prämix enthalten z. B. 10 mg Wirkstoff, 1 g DL-Methinoin, Rest Sojabohnenmehl.

Beispiel für die Zusammensetzung eines Schweineaufzuchtfutters, das erfindungsgemässe Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z. B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

Beispiel A
Ratten-Fütterungsversuch

Weibliche Laborratten 90–110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so dass Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so dass eine Vergleichbarkeit der Versuchsgruppen untereinander gewährleistet ist.

Während des 13tägigen Versuchs werden Gewichtszunahme und Futterverbrauch bestimmt.

Es werden die aus der Tabelle ersichtlichen Ergebnisse erhalten:

Tabelle: Ratten-Fütterungsversuch

| Wirkstoff Dosis 25 ppm | Gewichtszunahme |
|---|---|
| Kontrolle, ohne Wirkstoff | 100 |
| 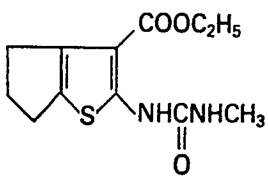 | 111 |
| 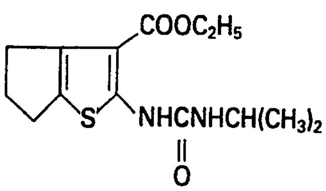 | 112 |
| 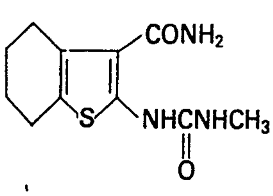 | 114 (10 ppm) |
| 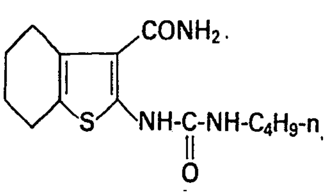 | 112 |

| Wirkstoff Dosis 25 ppm | Gewichtszunahme |
|---|---|
| 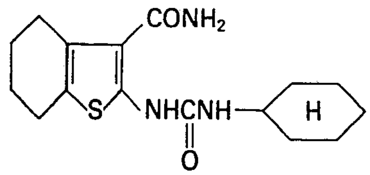 | 111 |
| 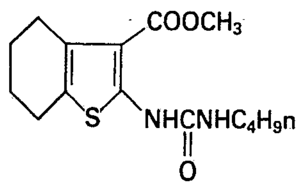 | 113 |
| 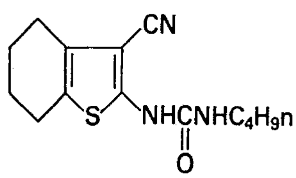 | 113 |
| 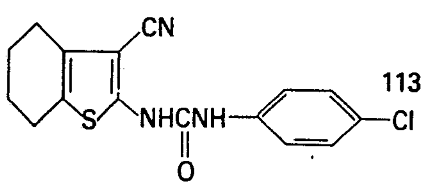 | 113 |
| 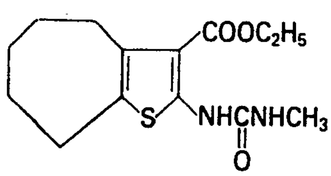 | 118 |
| 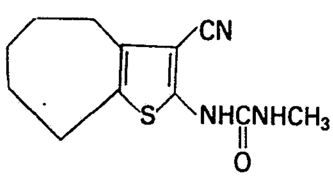 | 115 |
| 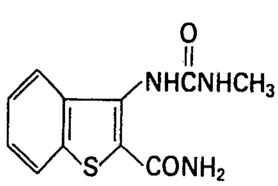 | 114 |

Herstellungsbeispiele

Beispiel 1
Herstellung von

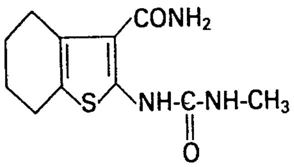

4,5 g (0,023 mol) 2-Amino-tetrahydrobenzothiophen-3-carbonsäureamid [hergestellt nach K. Gewald, Chem. Ber. 99, 94 (1966)] und 1,4 g (0,024 mol) Methylisocyanat wurden in 100 ml trockenem Chloroform 24 h unter Rückfluss erhitzt. Dann wurde die Chloroformphase dreimal mit je 50 ml Wasser gewaschen, über Natriumsulfat getrocknet und eingedampft. Das anfallende Rohprodukt wurde aus Ethanol umkristallisiert.

Ausbeute: 5,5 g (95%), Schmp. 202 °C (Zers.)
EA Ber. C 52,2 H 6,0 N 16,6
Gef. C 52,2 H 5,9 N 16,6

Beispiel 2
Herstellung von

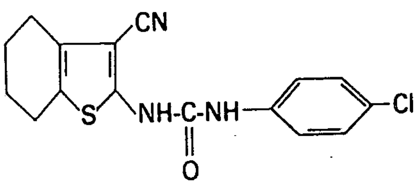

5,3 g (0,03 mol) 2-Amino-3-cyano-tetrahydrobenzothiophen [hergestellt nach K. Gewald, Chem. Ber. 99, 94 (1966)] und 5,1 g (0,033 mol) 4-Chlorphenylisocyanat wurden in 100 ml trockenem Pyridin 10 h bei 70 °C gerührt. Das ausgefallene Rohprodukt wurde abgesaugt, mit verdünnter Salzsäure und mit Wasser gewaschen und aus Ethanol umkristallisiert.

Ausbeute: 7,1 g (72%); Fp. > 250 °C.
EA Ber. C 57,9 H 4,3 N 12,7 Cl 10,7
Gef. C 58,0 H 4,2 N 12,7 Cl 10,7

Beispiel 3
N-Isopropyl-N'-2(3-cyan-4-tert.-butyl-thienyl)-harnstoff

Zu einer Lösung von 2,1 g (35,6 mmol) Isopropylamin, in 50 ml trockenem Toluol wurden 4 g (19,4 mmol) 2-Isocyanato-4-tert.-butyl-3-cyan-thiophen, gelöst in 50 ml trockenem Toluol, zugetropft. Es wurde eine Stunde bei Raumtemperatur gerührt. Zur Aufarbeitung wurde die Lösung in 1 l 2,5 N-Salzsäure eingerührt, die organische Phase abgetrennt und mit 100 ml $NaHCO_3$-Lösung gewaschen. Der nach Abdampfen des Toluols im Vakuum verbleibende Rückstand wurde aus Toluol/Petrolether umkristallisiert.

Ausbeute: 1,88 g (36,5% der Theorie),
Schmelzpunkt: 183–184 °C.

Beispiel 4
N-Isopropyl-N'-(2-carbomethoxy-thien-3-yl)harnstoff

Zu einer Lösung von 2,2 g (37 mmol) Isopropylamin in 50 ml trockenem Toluol wurde eine Lösung von 6,4 g (35 mmol) 2-Carbomethoxy-3-isocyanato-thiophen (Esso Research and Engineering Company, BE 767 244-Q) in 50 ml trockenem Toluol bei 0 °C langsam zugetropft. Das Produkt fiel als weisser Feststoff aus. Es wurde noch 2 h bei Raumtemperatur gerührt, dann abgesaugt und im Vakuum getrocknet.

Ausbeute: 6,8 g (80,3% der Theorie),
Schmelzpunkt: 119 °C.

Nach den Verfahren der Beispiele 1–4 wurden folgende Verbindungen erhalten:

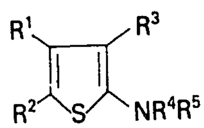

$R^4$ = H, $R^5$ = 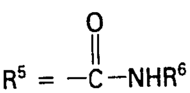

| Bsp.Nr. | $R^1$ | $R^2$ | $R^3$ | $R^6$ | Fp.[°C] |
|---|---|---|---|---|---|
| 5 | H | H | 3-$CO_2Et$ | 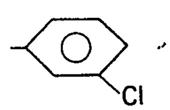 | 158 |
| 6 | H | H | 3-$CO_2Et$ | -$CH_3$ | 128 |
| 7 | H | H | 3-$CO_2Et$ | 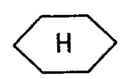 | 136 |
| 8 | H | H | 3-$CO_2Et$ | 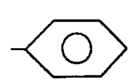 | 126 |

| Bsp.Nr. | $R^1$ | $R^2$ | $R^3$ | $R^6$ | Fp.[°C] |
|---|---|---|---|---|---|
| 9 | $-CH_3$ | $-CH_3$ | $3-CO_2Et$ | $-CH_3$ | 128 (Z.) |
| 10 | $-CH_3$ | $-CH_3$ | $3-CO_2Et$ | -n-Butyl | 78 |
| 11 | $-CH_3$ | $-CH_3$ | $3-CO_2Et$ | 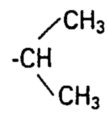 | 135 |
| 12 | $-CH_3$ | $-CH_3$ | $3-CO_2Et$ | 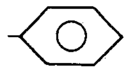 | 156 |
| 13 | H | H | $3-CO_2Et$ | 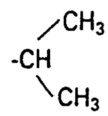 | 98 |
| 14 | 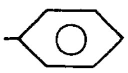 | H | $3-CO_2Et$ | $-CH_3$ | 131 |
| 15 | 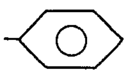 | H | $3-CO_2Et$ | 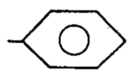 | 112–4 |
| 16 | 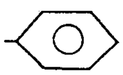 | H | $3-CO_2Et$ | 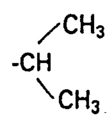 | 142 |
| 17 | H | 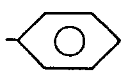 | $3-CO_2Et$ | $-CH_3$ | 145 |
| 18 | H | 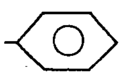 | $3-CO_2Et$ | n-Butyl | 122,5 |
| 19 | $-CH_3$ | $-CH_3$ | 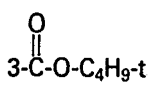 | $-CH_3$ | 159 |
| 20 | H | 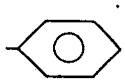 | 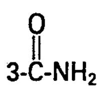 | $-CH_3$ | >250 |
| 21 | H | 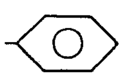 | 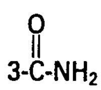 | 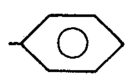 | >250 |
| 22 | H | 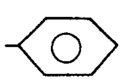 | 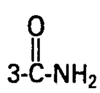 | 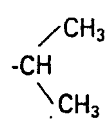 | >250 |
| 23 | H | 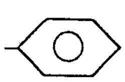 | $3-CO_2Et$ | 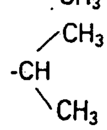 | 155 |

| Bsp.Nr. | $R^1$ | $R^2$ | $R^3$ | $R^6$ | Fp.[°C] |
|---|---|---|---|---|---|
| 24 | tert.-Butyl | H | 3-C≡N | H | 229 |
| 25 | H | i-Propyl | $3\text{-}CO_2Et$ | 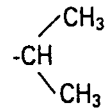 | 91 |
| 26 | tert.-Butyl | H | 3-C≡N | 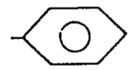 | 212,5 |
| 27 | H | 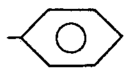 | $3\text{-}CO_2Et$ | H | 126,5 |
| 28 | $-C_2H_5$ | $-CH_3$ | $3\text{-}CO_2Et$ | $-CH_3$ | 121–2 |
| 29 | H | i-Propyl | $3\text{-}CO_2Et$ | 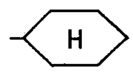 | 98–99 |
| 30 | H | H | $2\text{-}CO_2Me$ | 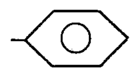 | 133 |
| 31 | H | H | $2\text{-}CO_2Me$ | H | 221 |
| 32 | H | H | $2\text{-}CO_2Me$ | $-CH_3$ | 139 |
| 33 | H | 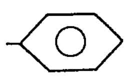 | $3\text{-}CO_2Et$ | 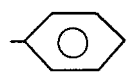 | 139–141 |
| 34 | -Et | $-CH_3$ | $3\text{-}CO_2Et$ | 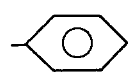 | 154 |
| 35 | -Et | $-CH_3$ | $3\text{-}CO_2Et$ | 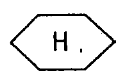 | 132–3 |
| 36 | -Et | $-CH_3$ | $3\text{-}CO_2Et$ | 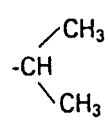 | 139–140 |
| 37 | -Et | $-CH_3$ | $3\text{-}CO_2Et$ | n-Butyl | 72 |
| 38 | $-CH_3$ | 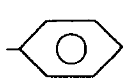 | 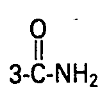 | $-CH_3$ | 222 |

| Bsp.Nr. | $R^1$ | $R^2$ | $R^3$ | $R^6$ | Fp.[°C] |
|---|---|---|---|---|---|
| 39 | $-CH_3$ | 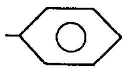 | 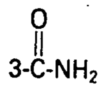 | 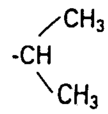 | 215 |
| 40 | $-CH_3$ | 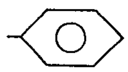 | 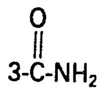 | 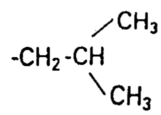 | 221 |
| 41 | $-CH_3$ | 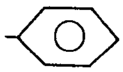 | 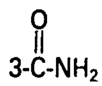 | -n-Butyl | 217 |
| 42 | $-CH_3$ | 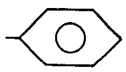 | 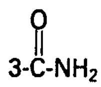 | 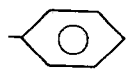 | >250 |
| 43 | H | H | $2-CO_2Me$ | 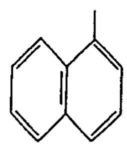 | 135 |
| 44 | H | H | 3-C≡N | 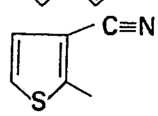 | 225 |
| 45 | H | H | $2-CO_2Me$ | n-Butyl | 72 |
| 46 | $-CH_3$ | 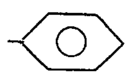 | $3-CO_2Et$ | $-CH_3$ | 135 |
| 47 | $-CH_3$ | 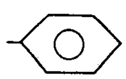 | $3-CO_2Et$ | n-Butyl | 119 |
| 48 | $-CH_3$ | 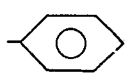 | $3-CO_2Et$ | 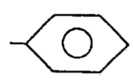 | 113 |
| 49 | $-CH_3$ | 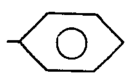 | $3-CO_2Et$ | 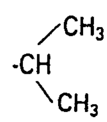 | 125 |
| 50 | $-(CH_2)_4$ | | 3-COOH | 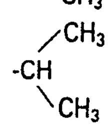 | 174 |

Weiter werden analog zu den Beispielen 1–4 Verbindungen der folgenden Formel erhalten:

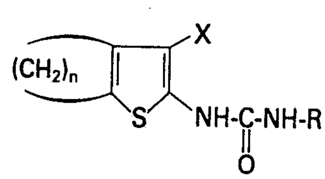

| Bsp.Nr. | n | X | R | Fp.[°C] |
|---|---|---|---|---|
| 51 | 3 | $COOC_2H_5$ | $CH_3$ | 165 |
| 52 | 3 | $COOC_2H_5$ | i-Propyl | 145 |
| 53 | 3 | $COOC_2H_5$ | 3-Chlorphenyl | 165 |
| 54 | 3 | CN | $-CH_3$ | 205 |
| 55 | 3 | CN | 4-Chlorphenyl | >270 |
| 56 | 4 | $COOCH_3$ | $CH_3$ | 167 |
| 57 | 4 | $COOCH_3$ | i-Propyl | 165 |
| 58 | 4 | $COOCH_3$ | n-Butyl | 130 |
| 59 | 4 | $COOCH_3$ | Phenyl | 176 |
| 60 | 4 | $COOC_4H_9t$ | $CH_3$ | 150 |
| 61 | 4 | $COCH_3$ | $CH_3$ | 193 |
| 62 | 4 | $COC_6H_5$ | Phenyl | 112 |
| 64 | 4 | $CONH_2$ | i-Propyl | 115 |
| 65 | 4 | $CONH_2$ | n-Butyl | 173 |
| 66 | 4 | $CONH_2$ | Cyclohexyl | 185 |
| 67 | 4 | $CONH_2$ | Phenyl | 200 |
| 68 | 4 | $CONH_2$ | 3-Chlorphenyl | 204 |
| 69 | 4 | $CONH_2$ | 4-Chlorphenyl | 221 |
| 70 | 4 | $CONHCH_3$ | $CH_3$ | 177 |
| 71 | 4 | CN | $CH_3$ | 209 |
| 72 | 4 | CN | i-Propyl | 217 |
| 73 | 4 | CN | n-Butyl | >260 |
| 74 | 4 | CN | Cyclohexyl | 225 |
| 75 | 4 | CN | Phenyl | 235 |
| 77 | 4 | CN | 2,6-Dichlorphenyl | >250 |
| 78 | 5 | $COOC_2H_5$ | $CH_3$ | 148 |
| 79 | 5 | $COOC_2H_5$ | i-Propyl | 113 |
| 80 | 5 | $COOC_2H_5$ | 3-Chlorphenyl | 98 |
| 81 | 5 | CN | $CH_3$ | 227 |
| 82 | 5 | CN | 4-Chlorphenyl | >250 |
| 83 | 5 | $CONH_2$ | $CH_3$ | >230 |

weiterhin wurden hergestellt:

| Bsp.Nr. | Formel | Fp[°C] |
|---|---|---|
| 84 | 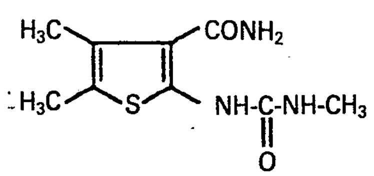 | 216 |
| 85 | 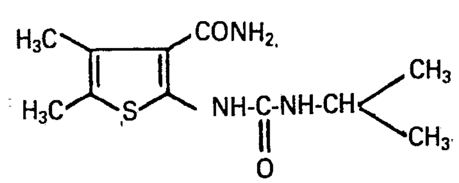 | >270 |
| 86 | 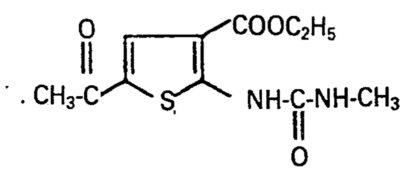 | 193 |

| Bsp.Nr. | Formel | Fp[°C] |
|---|---|---|
| 87 | 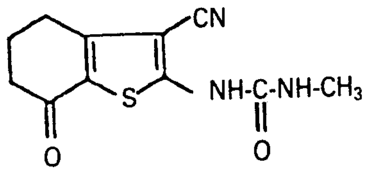 | >250 |
| 88 | 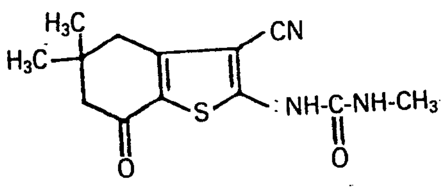 | 180 (Z.) |
| 89 | 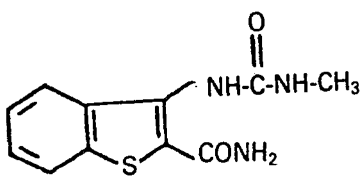 | 198 |
| 90 | 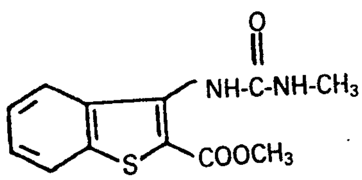 | >250 |

Weiterhin wurden hergestellt:

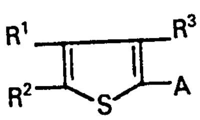

A = NH – $CONHR^6$

| Bsp.Nr. | $R^1$ | $R^2$ | $R^3$ | $R^6$ | Fp [°C] |
|---|---|---|---|---|---|
| 91 | H | i-Propyl | $CO_2Et$ | t-Butyl | 113–114 |
| 92 | H | i-Propyl | $CO_2Et$ | Phenyl | 121 |
| 93 | H | i-Propyl | $CO_2Et$ | 2-Butyl | 122 |
| 94 | H | Ethyl | $CO_2Et$ | i-Prop | 104 |
| 95 | H | Ethyl | $CO_2Et$ | 2-Butyl | 109 |
| 96 | H | Ethyl | $CO_2Et$ | Phenyl | 91 |
| 97 | H | i-Propyl | $CO_2Et$ | $CH_3$ | 84–86 |

| Bsp.Nr. | $R^1$ | $R^2$ | $R^3$ | $R^6$ | Fp [°C] |
|---|---|---|---|---|---|
| 98 | i-Propyl | H | $CONH_2$ | i-Prop | >250 |
| 99 | H | Ethyl | $CO_2Et$ | p-Tolyl | 97 |
| 100 | H | Ethyl | $CO_2Et$ | t-Butyl | 146 |
| 101 | Ethyl | $CH_3$ | $CO_2Et$ | p-Cl-Phenyl | 164 |
| 102 | Ethyl | $CH_3$ | $CO_2Et$ | m-Cl-Phenyl | 166 |
| 103 | Ethyl | $CH_3$ | $CO_2Et$ | p-$OCH_3$-Phenyl | 154 |
| 104 | Ethyl | $CH_3$ | $CO_2Et$ | p-Tolyl | 182 |
| 105 | Ethyl | $CH_3$ | $CO_2Et$ | p-$CF_3$-Phenyl | 177 |
| 106 | Ethyl | $CH_3$ | $CO_2Et$ | t-Butyl | 169 |
| 107 | Ethyl | $CH_3$ | $CO_2Et$ | o-Tolyl | 131 |
| 108 | Ethyl | $CH_3$ | $CO_2Et$ | o-$OCH_3$-Phenyl | 117 |
| 109 | Ethyl | $CH_3$ | $CO_2Et$ | 2-Butyl | 139 |
| 110 | $CH_3$ | Ethyl | $CO_2Et$ | o-Cl-Phenyl | 97 |
| 111 | $CH_3$ | Ethyl | $CO_2Et$ | m-Cl-Phenyl | 81 |
| 112 | $CH_3$ | Ethyl | $CO_2Et$ | p-Cl-Phenyl | 103 |
| 113 | $CH_3$ | Ethyl | $CO_2Et$ | p-$OCH_3$-Phenyl | 86 |
| 114 | $CH_3$ | Ethyl | $CO_2Et$ | p-Tolyl | 89 |
| 115 | $CH_3$ | Ethyl | $CO_2Et$ | p-$CF_3$-Phenyl | 97 |
| 116 | $CH_3$ | Ethyl | $CO_2Et$ | i-Propyl | 82 |
| 117 | $CH_3$ | Ethyl | $CO_2Et$ | Cyclohexyl | Öl |
| 118 | $EH_2$ | Ethyl | $CO_2Et$ | t-Butyl | 152 |
| 119 | $CH_3$ | Ethyl | $CO_2Et$ | Phenyl | 108 |
| 120 | $CH_3$ | Ethyl | $CO_2Et$ | o-Tolyl | 106 |
| 121 | $CH_3$ | Ethyl | $CO_2Et$ | o-$OCH_3$-Phenyl | Öl |
| 122 | $CH_3$ | Ethyl | $CO_2Et$ | 2-Butyl | Öl |
| 123 | H | $CH_3$ | $CO_2Et$ | o-Cl-Phenyl | 141 |
| 124 | H | $CH_3$ | $CO_2Et$ | m-Cl-Phenyl | 155 |
| 125 | H | $CH_3$ | $CO_2Et$ | p-Cl-Phenyl | 166 |
| 126 | H | $CH_3$ | $CO_2Et$ | p-$OCH_3$-Phenyl | 151 |
| 127 | H | $CH_3$ | $CO_2Et$ | p-Tolyl | 153 |

| Bsp.Nr. | $R^1$ | $R^2$ | $R^3$ | $R^6$ | Fp [°C] |
|---|---|---|---|---|---|
| 128 | H | $CH_3$ | $CO_2Et$ | m-$CF_3$-Phenyl | 156 |
| 129 | H | $CH_3$ | $CO_2Et$ | i-Propyl | 112 |
| 130 | H | $CH_3$ | $CO_2Et$ | Cyclohexyl | 122 |
| 131 | H | $CH_3$ | $CO_2Et$ | t-Butyl | 140 |
| 132 | H | $CH_3$ | $CO_2Et$ | Phenyl | 132 |
| 133 | H | $CH_3$ | $CO_2Et$ | o-$OCH_3$-Phenyl | 112 |
| 134 | H | $CH_3$ | $CO_2Et$ | o-Tolyl | 155 |
| 135 | H | $CH_3$ | $CO_2Et$ | 2-Butyl | 118 |
| 136 | H | $CH_3$ | $CO_2CH_3$ | 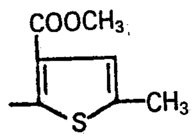 | 202 |
| 137 | H | n-Pent | $CO_2Et$ | $CH_3$ | 81 |
| 138 | H | Ethyl | $CO_2Et$ | Cyclohexyl | 101 |
| 139 | H | Ethyl | $CO_2Et$ | o-Cl-Phenyl | 108 |
| 140 | H | Ethyl | $CO_2Et$ | m-$CF_3$-Phenyl | 85 |
| 141 | H | Ethyl | $CO_2Et$ | o-Tolyl | 147 |
| 142 | H | Ethyl | $CO_2Et$ | o-$OCH_3$-Phenyl | 106 |
| 143 | H | Ethyl | $CO_2Et$ | m-Cl-Phenyl | 103 |
| 144 | H | Ethyl | $CO_2Et$ | p-Cl-Phenyl | 108 |
| 145 | H | $CH_3$ | $CO_2Et$ | $CH_3$ | 98 |
| 146 | Ethyl | $CH_3$ | $CO_2$-i-Propyl | t-Butyl | 183 |
| 147 | Ethyl | $CH_3$ | $CO_2$-i-Propyl | i-Butyl | 122 |
| 148 | Ethyl | $CH_3$ | $CO_2$-i-Propyl | i-Propyl | 175 |
| 149 | Ethyl | $CH_3$ | $CO_2$-i-Propyl | $CH_3$ | 130 |
| 150 | H | H | $CO_2Et$ | o-Cl-Phenyl | 137 |
| 151 | H | H | $CO_2Et$ | p-Cl-Phenyl | 171 |
| 152 | H | H | $CO_2Et$ | m-$CF_3$-Phenyl | 147 |
| 153 | H | H | $CO_2Et$ | 3,5-$Cl_2$-Phenyl | 189 |
| 154 | H | H | $CO_2Et$ | 3,4-$Cl_2$-Phenyl | 219 |
| 155 | H | H | $CO_2Et$ | p-Tolyl | 145 |

| Bsp.Nr. | $R^1$ | $R^2$ | $R^3$ | $R^6$ | Fp [°C] |
|---|---|---|---|---|---|
| 156 | H | H | $CO_2Et$ | p-$OCH_3$-Phenyl | 148 |
| 157 | H | H | $CO_2Et$ | p-$NO_2$-Phenyl | 240 |
| 158 | H | H | $CO_2Et$ | n-Butyl | 79 |
| 159 | H | H | $CO_2Et$ | t-Butyl | 176 |
| 160 | H | H | $CO_2Et$ | pF-Phenyl | 165 |
| 161 | H | H | $CO_2Et$ | Cyclohexyl | 137 |
| 162 | Ethyl | $CH_3$ | $CO_2Et$ | $CH_2CH_2$-S-$CH_3$ | Öl |
| 163 | H | H | $CO_2Et$ | o-$OCH_3$-Phenyl | 114 |
| 164 | H | i-Propyl | $CO_2Et$ | o-Cl-Phenyl | 112 |
| 165 | H | i-Propyl | $CO_2Et$ | m-Cl-Phenyl | 88 |
| 166 | H | i-Propyl | $CO_2Et$ | p-Cl-Phenyl | 135 |
| 167 | H | i-Propyl | $CO_2Et$ | p-$OCH_3$-Phenyl | 106 |
| 168 | H | i-Propyl | $CO_2Et$ | p-Tolyl | 108 |
| 169 | H | i-Propyl | $CO_2Et$ | m-$CF_3$-Phenyl | 122 |
| 170 | H | i-Propyl | $CO_2Et$ | o-Tolyl | 144 |
| 171 | H | i-Propyl | $CO_2Et$ | o-$OCH_3$-Phenyl | 111 |
| 172 | i-Propyl | H | $CONH_2$ | $CH_3$ | 195 |
| 173 | i-Propyl | H | $CONH_2$ | Phenyl | >250 |
| 174 | i-Propyl | H | $CONH_2$ | Cyclohexyl | 208 |
| 175 | H | H | $CO_2Et$ | 2,4-Dimethylphenyl | 176 |
| 176 | H | H | $CO_2Et$ | o-Tolyl | 142 |
| 177 | H | H | $CO_2Et$ | 3,5-Dimethoxyphenyl | 157 |
| 178 | H | H | $CO_2Et$ | 3,4-Dimethylphenyl | 151 |
| 179 | H | H | $CO_2Et$ | 3,4-Methylendioxyphenyl | 162 |
| 180 | H | H | $CO_2Et$ | m-Tolyl | 137 |
| 181 | H | H | $CO_2Et$ | 2,6-Dimethylphenyl | 109 |
| 182 | H | H | $CO_2Et$ | 2-$OCH_3$-4-$CH_3$-Phenyl | 132 |
| 183 | H | H | $CO_2Et$ | m-$OCH_3$-Phenyl | 143 |
| 184 | H | H | $CO_2Et$ | 2,5-Dimethoxyphenyl | 117 |
| 185 | H | H | $CO_2Et$ | 2,3-Dimethylphenyl | 176 |

| Bsp.Nr. | $R^1$ | $R^2$ | $R^3$ | $R^6$ | Fp [°C] |
|---|---|---|---|---|---|
| 186 | H | H | $CO_2Et$ | 3,5-Dimethylphenyl | 177 |
| 187 | H | H | $CO_2Et$ | 3,4-Dimethoxyphenyl | 165 |
| 188 | H | $CH_3$ | COOH | i-Propyl | 181 |
| 189 | H | $CH_3$ | COOH | o-Tolyl | 232 |
| 190 | H | Ethyl | $CO_2Et$ | $CH_3$ | 112 |
| 191 | $CH_3$ | H | $CO_2Et$ | i-Propyl | 121 |
| 192 | $CH_3$ | H | $CO_2Et$ | s-Butyl | 92 |
| 193 | $CH_3$ | H | $CO_2Et$ | 2-Butyl | 87 |
| 194 | $CH_3$ | H | $CO_2Et$ | t-Butyl | 137 |
| 195 | $CH_3$ | H | $CO_2Et$ | Cyclopentyl | 113 |
| 196 | $CH_3$ | H | $CO_1Et$ | Cyclohexyl | 163 |
| 197 | $CH_3$ | H | $CO_2Et$ | Phenyl | 147 |
| 198 | $CH_3$ | H | $CO_2Et$ | p-$OCH_3$-Phenyl | 108 |
| 199 | $CH_3$ | H | $CO_2Et$ | o-$OCH_3$-Phenyl | 94 |
| 200 | H | n-Pentyl | $CO_2Et$ | i-Propyl | Öl |
| 201 | H | n-Pentyl | $CO_2Et$ | s-Butyl | Öl |
| 202 | H | n-Pentyl | $CO_2Et$ | 2-Butyl | Öl |
| 203 | H | n-Pentyl | $CO_2Et$ | t-Butyl | 101 |
| 204 | H | n-Pentyl | $CO_2Et$ | Cyclohexyl | 73 |
| 205 | H | n-Pentyl | $CO_2Et$ | Phenyl | Öl |
| 206 | H | n-Pentyl | $CO_2Et$ | Cyclopentyl | 74 |
| 207 | H | n-Pentyl | $CO_2Et$ | p-$OCH_3$-Phenyl | 97 |
| 208 | H | n-Pentyl | $CO_2Et$ | o-$OCH_3$-Phenyl | Öl |
| 209 | H | n-Pentyl | $CO_2Et$ | A = $NHCONCH_3$-Phenyl | 48 |
| 210 | H | n-Pentyl | $CO_2Et$ | o-Tolyl | 80 |
| 211 | H | n-Pentyl | $CO_2Et$ | m-Tolyl | 65 |
| 212 | H | n-Pentyl | $CO_2Et$ | p-Tolyl | 93 |
| 213 | H | n-Pentyl | $CO_2Et$ | 2,3-Dimethylphenyl | 99 |
| 214 | H | n-Pentyl | $CO_2Et$ | 2-i-Propylphenyl | 73 |
| 215 | H | n-Pentyl | $CO_2Et$ | 2,4,5-Trimethylphenyl | 98 |

Weiterhin wurden hergestellt:

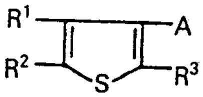

A = NHCONHR[6]

| Bsp.Nr. | $R^1$ | $R^2$ | $R^3$ | $R^6$ | Fp [°C] |
|---|---|---|---|---|---|
| 216 | $CO_2CH_3$ | H | $C_2H_5$ | $CH_3$ | 160 |
| 217 | $CO_2CH_3$ | H | $C_2H_5$ | i-Propyl | 166 |
| 218 | $CO_2CH_3$ | H | $C_2H_5$ | n-Butyl | 120 |

Herstellung der Ausgangsprodukte:

Beispiel Ia
2-Isocyanato-3-carboethoxythiophen

Zu 338 ml 20%iger Phosgenlösung in Toluol (0,68 mol) wurde bei −10 °C eine Lösung von 78 g (0,46 mol) 2-Amino-3-carbethoxythophen in 700 ml Toluol zugetropft. Nach beendetem Zutropfen liess man innerhalb einer Stunde auf Raumtemperatur kommen und erwärmte dann langsam während einer Stunde bis zum Sieden. Die nun dunkelbraune Lösung wurde noch 2 Stunden unter Rückfluss gekocht, danach das überschüssige Phosgen durch Einleiten eines trockenen Stickstoffs ausgetrieben. Anschliessend wurde das Toluol im Vakuum abdestilliert und mit dem Rückstand an der Ölpumpe destilliert.

Siedepunkt: 95 °C bei 6 Pa
Ausbeute: 61,8 g, 69% der Theorie
Ausgangssubstanzen:
K. Gewald, Chem. Ber. 98, 3571–3577 (1965)
K. Gewald, E. Schinke und H. Böttcher, Chem. Ber. 99, 94–100 (1966).

Analog erhielt man die Thienylisocyanate der Formel III

Analog wurden erhalten:

Ib 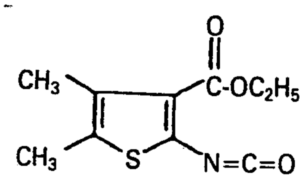 Schmp.: 38 °C

Ic 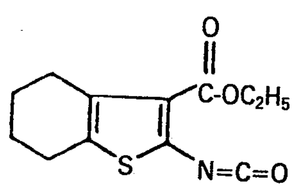 Sdp.: 120 °C (1 Pa)

Id 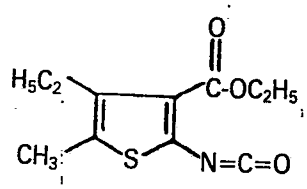 Sdp.: 101 °C (30 Pa)

Ie 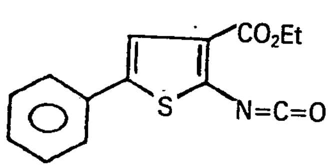 Schmp.: 90–93 °C

If

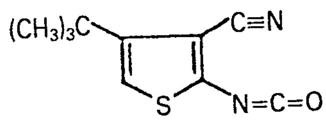

Schmp.: 62–63 °C

Ig

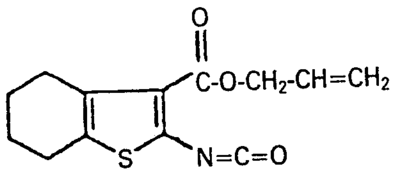

Sdp.: 160 °C (30 Pa)
IR: 2200, 1690 $cm^{-1}$.
im Kugelrohr destilliert

Ih

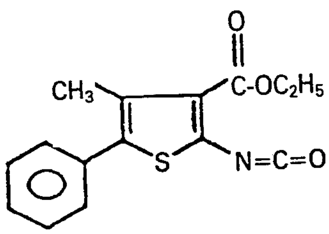

Sdp.: 142–147 °C (5 Pa)
IR: 2250, 1690 $cm^{-1}$

Ii

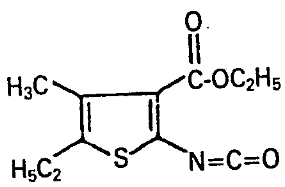

Sdp.: 103 °C (30 Pa)
IR: 2250, 1690 $cm^{-1}$

Ij

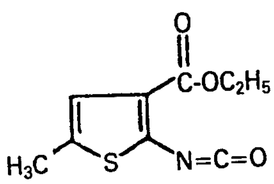

Sdp.: 88 °C (20 Pa)
IR: 2250, 1700
Schmp.: 45 °C

Ik

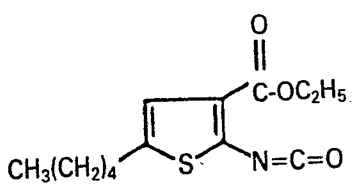

Sdp.: 125 °C (90 Pa)
IR: 2250, 1710

Il

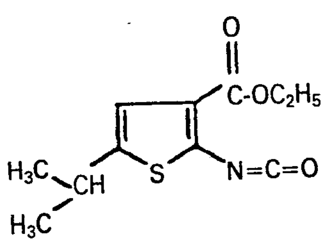

Sdp.: 96 °C (15 Pa)
IR: 2250, 1710

Im

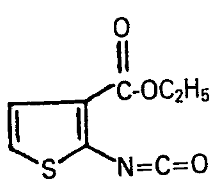

Sdp.: 75 °C (40 Pa)

In 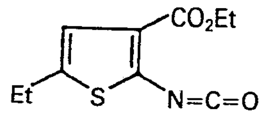

Sdp.: 105 °C (20 Pa)

Beispiel IIa

2-Amino-3-t-butyloxycarbonyl-4,5-dimethylthiophen

Ansatz: 100 g (0,71 mol) Cyanessigsäure-tert.-butylester
51,2 g (0,71 mol) Butanon
23,9 g (0,75 mol) Schwefel
71 ml Morpholin
140 ml Ethanol p.A.

Das Keton wurde in Ethanol gelöst, dann wurden Morpholin und Schwefel zugegeben.

Zu der gelben Suspension wurde Cyanessigsäure-tert.-butylester zugetropft. Anschliessend wurden 3 h auf 60 °C erwärmt. Nach Abkühlung wurde das Gemisch auf 1 l Wasser gegossen, 750 ml Ether zugefügt, die organische Phase abgetrennt, die wässrige Phase mit 200 ml Ether extrahiert.

Die vereinigten Extrakte wurden mit 2×200 ml NaOH (5%ig), 200 ml Wasser, 2×200 ml 5%iger $H_2SO_4$, 200 ml Wasser und 200 ml $NaHCO_3$ gewaschen, mit $Na_2SO_4$ getrocknet. Nach Verdampfen des Lösungsmittels im Vakuum verblieben 133,8 g.

Impfkristalle wurden zum Rohprodukt gegeben, wobei der Kolbeninhalt erstarrte.

Ausbeute: 50 g = 31% der Theorie

Fp: 82–85 °C

Analog erhält man die Aminothiophene der Formel

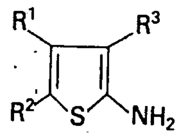

| Bsp.Nr. | $R^1$ | $R^2$ | $R^3$ | Physik. Daten |
|---|---|---|---|---|
| IIb | $C_2H_5$ | $CH_3$ | $COOC_2H_5$ | Fp 44 °C |
| IIc | H | i-Propyl | $COOC_2H_5$ | 101 °C (5 Pascal) |
| IId | H | i-Butyl | $COOC_2H_5$ | |
| IIe | H | n-Pentyl | $COOC_2H_5$ | 152 °C (50 Pascal) |
| IIf | $CH_3$ | $C_2H_5$ | $COOC_2H_5$ | 148 °C (250 Pascal) |

| Bsp.Nr. | $R^1$ $R^2$ | $R^3$ | Fp [°C] |
|---|---|---|---|
| IIg | $-(-CH_2-)-_3$ | $COO_2CH_5$ | 90 |
| IIh | $-(-CH_2-)-_3$ | CN | 149 |
| IIi | $-(-CH_2-)-_4$ | $COOCH_3$ | 112 |
| IIj | $-(-CH_2-)-_4$ | CN | 143 |
| IIk | $-(-CH_2-)-_4$ | $CONH_2$ | 185 |
| IIl | $-(-CH_2-)-_5$ | $COOC_2H_5$ | 105 |
| IIm | $-(-CH_2-)-_5$ | CN | 121 |
| IIn | $-(-CH_2-)-_5$ | $CONH_2$ | 170 |

## Patentansprüche

1. Verwendung von Thienylharnstoffen oder -isoharnstoffen der Formel I

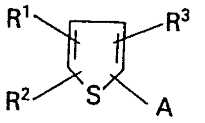 I

in welcher

A für die Reste Ia und Ib steht

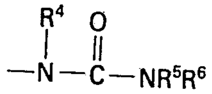 Ia

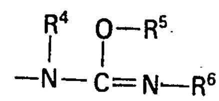 Ib

$R^1$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Alkyl, Acyl, Aroyl, Aryl steht,

$R^2$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Acyl, Aroyl, Alkyl, Aryl steht,

$R^1$ und $R^2$ gemeinsam mit den angrenzenden C-Atomen für einen gegebenenfalls substituierten gesättigten oder ungesättigten carbocyclischen oder heterocyclischen Ring stehen, der gegebenenfalls eine Carbonylfunktion tragen kann,

$R^3$ für die Reste CN, $COOR^7$, $CONR^8R^9$, $COR^{10}$ steht,

$R^4$ für Wasserstoff oder Alkyl steht,

$R^5$ für Wasserstoff, gegebenenfalls substituiertes Alkyl, Cycloalkyl, Alkenyl, gegebenenfalls substituiertes Aryl oder Heteroaryl steht,

$R^6$ für Wasserstoff, gegebenenfalls substituiertes Alkyl, Cycloalkyl, Alkenyl, gegebenenfalls substituiertes Aryl oder Heteroaryl steht,

$R^7$ für Wasserstoff, gegebenenfalls substituiertes Alkyl, Cycloalkyl, Alkenyl, gegebenenfalls substituiertes Aryl steht,

$R^8$ für Wasserstoff, Alkyl oder Cycloalkyl steht,

$R^9$ für Wasserstoff, gegebenenfalls substituiertes Alkyl, gegebenenfalls substituiertes Aryl steht,

$R^{10}$ für gegebenenfalls substituiertes Alkyl, gegebenenfalls substituiertes Aryl steht,

als leistungsfördernde Mittel für Tiere.

2. Thienylisocyanate der Formel III

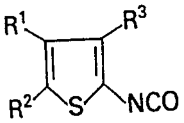

III

in welcher

$R^1$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Alkyl, Acyl, Aroyl, Aryl steht,

$R^2$ für Wasserstoff, Halogen, Nitro, CN, Alkoxy, Alkylthio, Halogenalkoxy, Halogenalkylthio, Alkoxyalkyl, gegebenenfalls substituierte Reste aus der Gruppe Acyl, Aroyl, Alkyl, Aryl steht,

$R^1$ und $R^2$ gemeinsam mit den angrenzenden C-Atomen für einen gegebenenfalls substituierten gesättigten oder ungesättigten carbocyclischen Ring stehen, der gegebenenfalls eine Carbonylfunktion tragen kann,

$R^3$ für die Reste $COOR^7$, $CONR^8R^9$, $COR^{10}$ steht,

$R^7$ für Wasserstoff, gegebenenfalls substituiertes Methyl, Cycloalkyl, $C_{2-4}$-Alkenyl, gegebenenfalls substituiertes Aryl steht,

$R^8$ für Wasserstoff, Alkyl oder Cycloalkyl steht,

$R^9$ für Wasserstoff, gegebenenfalls substituiertes Alkyl, gegebenenfalls substituiertes Aryl steht,

$R^{10}$ für gegebenenfalls substituiertes Alkyl, gegebenenfalls substituiertes Aryl steht mit Ausnahme von 3-Methoxycarbonyl-thien-2-yl-isocyanat.

3. Verfahren zur Herstellung der Thienylisocyanate der Formel III gemäss Anspruch 2, dadurch gekennzeichnet, dass man Thienylamine der Formel V

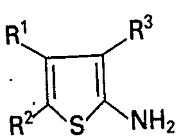

V

in welcher

$R^1$, $R^2$, $R^3$ die in Anspruch 2 angegebene Bedeutung besitzen,

mit Phosgen umsetzt.

4. Thienylharnstoffe oder -isoharnstoffe der Formel VI

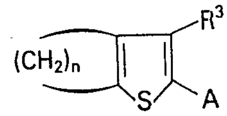

VI

in welcher

n für 3, 4, 5 oder 6 steht,

A für die Reste Ia und Ib steht

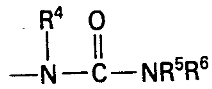

Ia

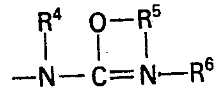

. Ib

$R^3$ für den Fall, dass n für 3, 5, 6 steht, für die Reste CN, $COOR^7$, $CONR^8R^9$, $COR^{10}$ steht und für den Fall, dass n für 4 steht, für die Reste $COOCH_3$, $COO(C_{2-4}$-Alkenyl), $CONR^8R^9$, $COR^{10}$ steht,

$R^4$ für Wasserstoff oder Alkyl steht,

$R^5$ für Wasserstoff, gegebenenfalls substituiertes Alkyl, Cycloalkyl, Alkenyl, gegebenenfalls substituiertes Aryl steht,

$R^6$ für Wasserstoff, gegebenenfalls substituiertes Alkyl, Cycloalkyl, Alkenyl, gegebenenfalls substituiertes Aryl steht,

$R^7$ für Wasserstoff, gegebenenfalls substituiertes Alkyl, Cycloalkyl, Alkenyl, gegebenenfalls substituiertes Aryl steht,

$R^8$ für Wasserstoff, Alkyl oder Cycloalkyl steht,

$R^9$ für Wasserstoff, gegebenenfalls substituiertes Alkyl, gegebenenfalls substituiertes Aryl steht,

$R^{10}$ für gegebenenfalls substituiertes Alkyl, gegebenenfalls substituiertes Aryl steht.

5. Verfahren zur Herstellung der Thienylharnstoffe oder -isoharnstoffe der Formel VI

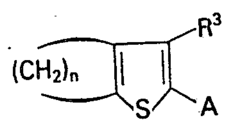

VI

in welcher

n für 3, 4 oder 6 steht,

A für die Reste Ia und Ib steht

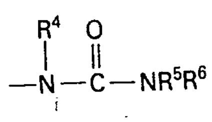

Ia

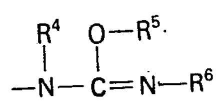

. Ib

$R^3$ für den Fall, dass n für 4, 5, 6 steht, für die Reste CN, $COOR^7$, $CONR^8R^9$, $COR^{10}$ steht und für den Fall, dass n für 4 steht, für die Reste $COOCH_3$, $COO(C_{2-4}$-Alkenyl), $CONR^8R^9$, $COR^{10}$ steht,

$R^4$ für Wasserstoff oder Alkyl steht,

$R^5$ für Wasserstoff, gegebenenfalls substituiertes Alkyl, Cycloalkyl, Alkenyl, gegebenenfalls substituiertes Aryl oder Heteroaryl steht,

$R^6$ für Wasserstoff, gegebenenfalls substituiertes Alkyl, Cycloalkyl, Alkenyl, gegebenenfalls substituiertes Aryl oder Heteroaryl steht,

$R^7$ für Wasserstoff, gegebenenfalls substituiertes Alkyl, Cycloalkyl, Alkenyl, gegebenenfalls substituiertes Aryl steht,

$R^8$ für Wasserstoff, Alkyl oder Cycloalkyl steht,

$R^9$ für Wasserstoff, gegebenenfalls substituiertes Alkyl, gegebenenfalls substituiertes Aryl steht,

$R^{10}$ für gegebenenfalls substituiertes Alkyl, gegebenenfalls substituiertes Aryl steht,

dadurch gekennzeichnet,

a) dass man für den Fall, dass A für den Rest Ia steht und $R^5$ für Wasserstoff steht, Thienylamine der Formel VII

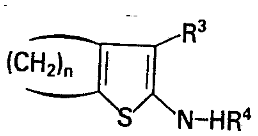

VII

in welcher

n, $R^3$ und $R^4$ die oben angegebene Bedeutung haben,

mit Isocyanaten der Formel VIII

$OCN-R^6$ VIII

in welcher

$R^6$ die oben angegebene Bedeutung hat,

umsetzt, oder

b) dass man für den Fall, dass A für den Rest Ia steht und $R^4$ für Wasserstoff steht, Thienylisocyanate der Formel IX

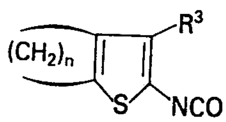

IX

in welcher

n und $R^3$ die oben angegebene Bedeutung haben,

mit Aminen der Formel IV

$H-NR^5R^6$ , IV

in welcher

$R^5$ und $R^6$ die oben angegebene Bedeutung haben,

umsetzt, oder

c) dass man für den Fall, dass A für den Rest Ib steht, Thienylamine der Formel VII

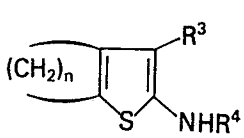

VII

in welcher

n, $R^3$ und $R^4$ die oben angegebene Bedeutung haben,

mit Imidokohlensäureesterhalogeniden der Formel X

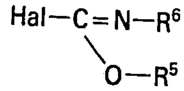

X

in welcher

$R^5$ und $R^6$ die oben angegebene Bedeutung haben und

Hal für Halogen steht,

umsetzt.

6. Mittel zur Leistungsförderung von Tieren, gekennzeichnet durch einen Gehalt an Thienylharnstoffen oder -isoharnstoffen der Formel I gemäss Anspruch 1.

7. Tierfutter, Trinkwasser für Tiere, Zusätze für Tierfutter und Trinkwasser für Tiere, gekennzeichnet durch einen Gehalt an Thienylharnstoffen oder -isoharnstoffen der Formel I gemäss Anspruch 1.

8. Verwendung von Thienylharnstoffen oder -isoharnstoffen der Formel 1 gemäss Anspruch 1 zur Leistungsförderung von Tieren.

9. Verfahren zur Herstellung von Mitteln zur Leistungsförderung von Tieren, dadurch gekennzeichnet, dass man Thienylharnstoffe oder -isoharnstoffe der Formel I gemäss Anspruch 1 mit Streck- und/oder Verdünnungsmitteln vermischt.

10. Verfahren zur Herstellung von Tierfutter, Trinkwasser für Tiere oder Zusätze für Tierfutter und Trinkwasser für Tiere, dadurch gekennzeichnet, dass man Thienylharnstoffe oder -isoharnstoffe der Formel I gemäss Anspruch 1 mit Futtermitteln oder Trinkwasser und gegebenenfalls weiteren Hilfsstoffen vermischt.

## Claims

1. Use of thienylureas or -isoureas of the formula I

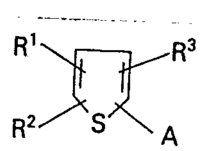

I

in which

A represents the radicals Ia and Ib

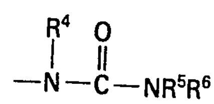

Ia

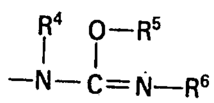

Ib

$R^1$ represents hydrogen, halogen, nitro, CN, alkoxy, alkylthio, halogenoalkoxy, halogenalkylthio, alkoxyalkyl or optionally substituted radicals from the group comprising alkyl, acyl, aroyl and aryl,

$R^2$ represents hydrogen, halogen, nitro, CN, alkoxy, alkylthio, halogenoalkoxy, halogenoalkylthio, alkoxyalkyl or optionally substituted radicals from the group comprising acyl, aroyl, alkyl and aryl, or
$R^1$ and $R^2$, together with the adjacent C atoms, represent an optionally substituted saturated or unsaturated carbocyclic or heterocyclic ring, which can optionally carry a carbonyl function,
$R^3$ represents the radicals CN, $COOR^7$, $CONR^8R^9$ or $COR^{10}$,
$R^4$ represents hydrogen or alkyl,
$R^5$ represents hydrogen, optionally substituted alkyl, cycloalkyl, alkenyl, optionally substituted aryl or heteroaryl,
$R^6$ represents hydrogen, optionally substituted alkyl, cycloalkyl, alkenyl, optionally substituted aryl or heteroaryl,
$R^7$ represents hydrogen, optionally substituted alkyl, cycloalkyl, alkenyl or optionally substituted aryl,
$R^8$ represents hydrogen, alkyl or cycloalkyl,
$R^9$ represents hydrogen, optionally substituted alkyl or optionally substituted aryl and
$R^{10}$ represents optionally substituted alkyl or optionally substituted aryl,
as growth-promoting agents for animals

2. Thienyl isocyanates of the formula III

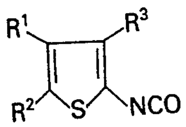

III

in which
$R^1$ represents hydrogen, halogen, nitro, CN, alkoxy, alkylthio, halogenoalkoxy, halogenoalkylthio, alkoxyalkyl or optionally substituted radicals from the group comprising alkyl, acyl, aroyl and aryl,
$R^2$ represents hydrogen, halogen, nitro, CN, alkoxy, alkylthio, halogenoalkoxy, halogenoalkylthio, alkoxyalkyl or optionally substituted radicals from the group comprising acyl, aroyl, alkyl and aryl, or
$R^1$ and $R^2$, together with the adjacent C atoms, represent an optionally substituted saturated or unsaturated carbocyclic ring, which can optionally carry a carbonyl function,
$R^3$ represents the radicals $COOR^7$, $CONR^8R^9$ or $COR^{10}$,
$R^7$ represents hydrogen, optionally substituted methyl, cycloalkyl, $C_{2-4}$-alkenyl or optionally substituted aryl,
$R^8$ represents hydrogen, alkyl or cycloalkyl,
$R^9$ represents hydrogen, optionally substituted alkyl or optionally substituted aryl and
$R^{10}$ represents optionally substituted alkyl or optionally substituted aryl,
with the exception of 3-methoxycarbonylthien-2-yl-isocyanate.

3. Process for the preparation of the thienyl isocyanates of the formula III according to claim 2, characterised in that thienylamines of the formula V

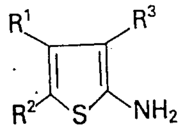

V

in which
$R^1$, $R^2$ and $R^3$ have the meaning stated in claim 2,
are reacted with phosgene.

4. Thienylureas or -isoureas of the formula VI

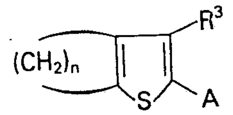

VI

in which
n represents 3, 4, 5 or 6,
A represents the radicals Ia and Ib

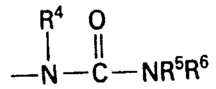

Ia

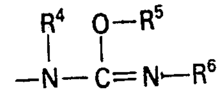

Ib

$R^3$, in the case where n represents 3, 5 or 6, represents the radicals CN, $COOR^7$, $CONR^8R^9$ or $COR^{10}$, or, in the case where n represents 4, represents the radicals $COOCH_3$, $COO(C_{2-4}\text{-alkenyl})$, $CONR^8R^9$ or $COR^{10}$,
$R^4$ represents hydrogen or alkyl,
$R^5$ represents hydrogen, optionally substituted alkyl, cycloalkyl, alkenyl or optionally substituted aryl,
$R^6$ represents hydrogen, optionally substituted alkyl, cycloalkyl, alkenyl or optionally substituted aryl,
$R^7$ represents hydrogen, optionally substituted alkyl, cycloalkyl, alkenyl or optionally substituted aryl,
$R^8$ represents hydrogen, alkyl or cycloalkyl,
$R^9$ represents hydrogen, optionally substituted alkyl or optionally sbstituted aryl and
$R^{10}$ represents optionally substituted alkyl or optionally substituted aryl.

5. Process for the preparation of the thienylureas or -isoureas of the formula VI

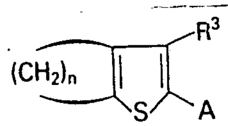

VI

in which
n represents 3, 4, 5 or 6,
A represents the radicals Ia and Ib

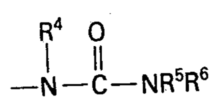

Ia

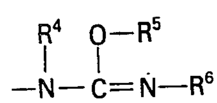

Ib

$R^3$, in the case where n represents 4, 5, or 6, represents the radicals CN, $COOR^7$, $CONR^8R^9$ or $COR^{10}$, or, in the case where n represents 4, represents the radicals $COOCH_3$, $COO(C_{2-4}\text{-alkenyl})$, $CONR^8R^9$ or $COR^{10}$,

$R^4$ represents hydrogen or alkyl,

$R^5$ represents hydrogen, optionally substituted alkyl, cycloalkyl, alkenyl, optionally substituted aryl or heteroaryl,

$R^6$ represents hydrogen, optionally substituted alkyl, cycloalkyl, alkenyl, optionally substituted aryl or heteroaryl,

$R^7$ represents hydrogen, optionally substituted alkyl, cycloalkyl, alkenyl or optionally substituted aryl,

$R^8$ represents hydrogen, alkyl or cycloalkyl,

$R^9$ represents hydrogen, optionally substituted alkyl or optionally substituted aryl and

$R^{10}$ represents optionally substituted alkyl or optionally substituted aryl,

characterised in that

a) in the case where A represents the radical Ia and $R^5$ represents hydrogen, thienylamines of the formula VII

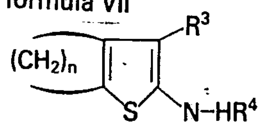 VII

in which

n, $R^3$ and $R^4$ have the abovementioned meaning, are reacted with isocyanates of the formula VIII

$OCN-R^6$ VIII

in which

$R^6$ has the abovementioned meaning,

or

b) in the case where A represents the radical Ia and $R^4$ represents hydrogen, thienyl isocyanates of the formula IX

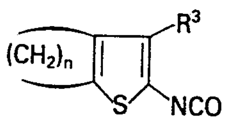 IX

in which

n and $R^3$ have the abovementioned meaning, are reacted with amines of the formula IV

$H-NR^5R^6$ IV

in which

$R^5$ and $R^6$ have the abovementioned meaning,

or

c) in the case where A represents the radical Ib, thienylamines of the formula VII

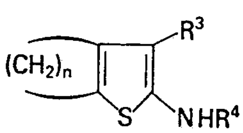 VII

in which

n, $R^3$ and $R^4$ have the abovementioned meaning, are reacted with imidocarbonic acid ester-halides of the formula X

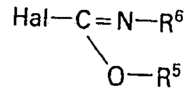 X

in which

$R^5$ and $R^6$ have the abovementioned meaning and

Hal represents halogen.

6. Agents for promoting growth in animals, containing thienylureas or -isoureas of the formula I according to claim 1.

7. Animal feed, drinking water for animals, additives for animal feed and drinking water for animals, containing thienylureas or -isoureas of the formula I according to claim 1.

8. Use of thienylureas or -isoureas of the formula I, according to claim 1 for growth promition in animals.

9. Process for the preparation of agents for promoting growth in animals, characterised in that thienylureas or -isoureas of the formula I according to claim 1 are mixed with extenders and/or diluents.

10. Process for the preparation of animals feed, drinking water for animals or additives for animal feed and drinking water for animals, characterised in that thienylureas or -isoureas of the formula I according to claim 1 are mixed with feedstuffs or drinking water and, if appropriate, further auxiliaries.

## Revendications

1. Utilisation de thiényl-urées ou de thiényl-isourées de formule I

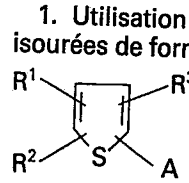 I

dans laquelle

A représente les radicaux Ia et Ib

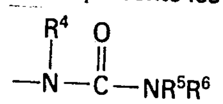 Ia

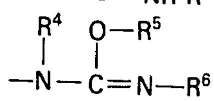 Ib

$R^1$ représente un atome d'hydrogène, un atome d'halogène, un groupe nitro, un groupe CN, un groupe alcoxy, un groupe alkylthio, un groupe halogénalcoxy, un groupe halogénalkylthio, un groupe alcoxyalkyle, ainsi que des radicaux éventuellement substitués choisis parmi le groupe comprenant un groupe alkyle, un groupe acyle, un groupe aroyle, un groupe aryle,

$R^2$ représente un atome d'hydrogène, un atome d'halogène, un groupe nitro, un groupe CN, un groupe alcoxy, un groupe alkylthio, un groupe halogénalkoxy, un groupe halogénalkylthio, un groupe alcoxyalkyle, des radicaux éventuellement substitués choisis parmi le groupe comprenant un groupe acyle, un groupe aroyle, un groupe alkyle ou un groupe aryle,

$R^1$ et $R^2$, ensemble avec les atomes de carbone adjacents, représentent un noyau hétérocyclique ou carbocyclique saturé ou insaturé et éventuellement substitué pouvant éventuellement comporter une fonction carbonyle,

$R^3$ représente les radicaux CN, $COOR^7$, $CONR^8R^9$, $COR^{10}$,

$R^4$ représente un atome d'hydrogène ou un groupe alkyle,

$R^5$ représente un atome d'hydrogène, un groupe alcényle, un groupe cycloalkyle, un groupe alkyle éventuellement substitué, un groupe hétéroaryle ou un groupe aryle éventuellement substitué,

$R^6$ représente un atome d'hydrogène, un groupe alcényle, un groupe cycloalkyle ou un groupe alkyle éventuellement substitué, un groupe hétéroaryle ou un groupe aryle éventuellement substitué,

$R^7$ représente un atome d'hydrogène, un groupe alcényle, un groupe cycloalkyle ou un groupe alkyle éventuellement substitué, ou un groupe aryle éventuellement substitué,

$R^8$ représente un atome d'hydrogène, un groupe alkyle ou un groupe cycloalkyle,

$R^9$ représente un atome d'hydrogène, un groupe alkyle éventuellement substitué, ou un groupe aryle éventuellement substitué,

$R^{10}$ représente un groupe alkyle éventuellement substitué, ou un groupe aryle éventuellement substitué,

comme agents favorisant le rendement chez les animaux.

2. Thiénylisocyanates de formule III

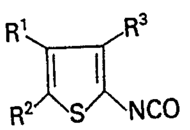

III

dans laquelle

$R^1$ représente un atome d'hydrogène, un atome d'halogène, un groupe nitro, un groupe CN, un groupe alcoxy, un groupe alkylthio, un groupe halogénalcoxy, un groupe halogénalkylthio, un groupe alcoxyalkyle, ou des radicaux éventuellement substitués choisis parmi le groupe comprenant un groupe alkyle, un groupe acyle, un groupe aroyle ou un groupe aryle,

$R^2$ représente un atome d'hydrogène, un atome d'halogène, un groupe nitro, un groupe CN, un groupe alcoxy, un groupe alkylthio, un groupe halogénalcoxy, un groupe halogénalkylthio, un groupe alcoxyalkyle, ou des radicaux éventuellement substitués choisis parmi le groupe comprenant un groupe acyle, un groupe aroyle, un groupe alkyle ou un groupe aryle,

$R^1$ et $R^2$, ensemble avec les atomes de carbone adjacents, représentent un noyau carbocyclique saturé ou insaturé éventuellement substitué pouvant éventuellement comporter une fonction carbonyle,

$R^3$ représente les radicaux $COOR^7$, $CONR^8R^9$, $COR^{10}$,

$R^7$ représente un atome d'hydrogène, un groupe alcényle en $C_2$-$C_4$, un groupe cycloalkyle, ou un groupe méthyle éventuellement substitué, ou encore un groupe aryle éventuellement substitué,

$R^8$ représente un atome d'hydrogène, un groupe alkyle ou un groupe cycloalkyle,

$R^9$ représente un atome d'hydrogène, un groupe alkyle éventuellement substitué, ou un groupe aryle éventuellement substitué,

$R^{10}$ représente un goupe alkyle éventuellement substitué, ou un groupe aryle éventuellement substitué, à l'exception du 3-méthoxycarbonyl-thién-2-yl-isocyanate.

3. Procédé de préparation de thiénylisocyanates de formule III selon la revendication 2, caractérisé en ce qu'on fait réagir des thiénylamines de formule V

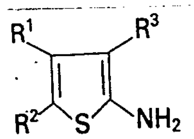

V

dans laquelle

$R^1$, $R^2$ et $R^3$ ont les significations indiquées dans la revendication 2,

avec du phosgène.

4. Thiénylurées ou thiénylisourées de formule VI

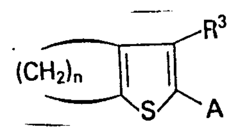

VI

dans laquelle

n représente 3, 4, 5 ou 6,

A représente les radicaux Ia et Ib

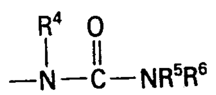

Ia

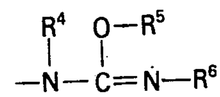

Ib

$R^3$ représente, dans le cas où n est égal à 3, 5 ou 6, les radicaux CN, $COOR^7$, $CONR^8R^9$, $COR^{10}$ et, dans le cas où n est égal à 4, il représente les radicaux $COOCH_3$, COO(alcényle en $C_2$-$C_4$), $CONR^8R^9$, $COR^{10}$,

$R^4$ représente un atome d'hydrogène ou un groupe alkyle,

$R^5$ représente un atome d'hydrogène, un groupe alcényle, un groupe cycloalkyle, ou un groupe alkyle éventuellement substitué, ou encore un groupe aryle éventuellement substitué,

$R^6$ représente un atome d'hydrogène, un groupe alcényle, un groupe cycloalkyle ou un groupe alkyle éventuellement substitué, ou encore un groupe aryle éventuellement substitué,

$R^7$ représente un atome d'hydrogène, un groupe alcényle, un groupe cycloalkyle, ou un groupe alkyle éventuellement substitué, ou encore un groupe aryle éventuellement substitué,

$R^8$ représente un atome d'hydrogène, un groupe alkyle ou un groupe cycloalkyle,

$R^9$ représente un atome d'hydrogène, un groupe alkyle éventuellement substitué, ou un groupe aryle éventuellement substitué,

$R^{10}$ représente un groupe alkyle éventuellement substitué, ou un groupe aryle éventuellement substitué.

5. Procédé de préparation de thiénylurées ou de thiénylisourées de formule VI

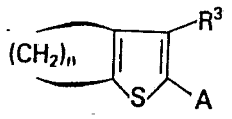

VI

dans laquelle

n représente 3, 4 ou 6,

A représente les radicaux Ia et Ib

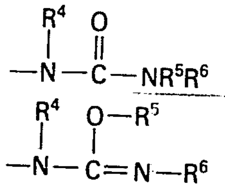

Ia

Ib

$R^3$ représente, dans le cas où n est égal à 4, 5 ou 6, les radicaux CN, $COOR^7$, $CONR^8R^9$, $COR^{10}$ et, dans le cas où n est égal à 4, il représente les radicaux $COOCH_3$, COO(alcényle en $C_2$-$C_4$), $CONR^8R^9$, $COR^{10}$,

$R^4$ représente un atome d'hydrogène ou un groupe alkyle,

$R^5$ représente un atome d'hydrogène, un groupe alcényle, un groupe cycloalkyle ou un groupe alkyle éventuellement substitué, ou encore un groupe hétéroaryle ou un groupe aryle éventuellement substitué,

$R^6$ représente un atome d'hydrogène, un groupe alcényle, un groupe cycloalkyle, un groupe alkyle éventuellement substitué, un groupe hétéroaryle ou un groupe aryle éventuellement substitué,

$R^7$ représente un atome d'hydrogène, un groupe alcényle, un groupe cycloalkyle, ou un groupe alkyle éventuellement substitué, ou encore un groupe aryle éventuellement substitué,

$R^8$ représente un atome d'hydrogène, un groupe alkyle ou un groupe cycloalkyle,

$R^9$ représente un atome d'hydrogène, un groupe alkyle éventuellement substitué, ou un groupe aryle éventuellement substitué,

$R^{10}$ représente un groupe alkyle éventuellement substitué ou un groupe aryle éventuellement substitué,

caractérisé en ce que,

a) dans le cas où A représente le radical Ia et $R^5$ représente un atome d'hydrogène, on fait réagir des thiénylamines de formule VII

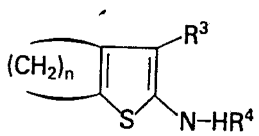

VII

dans laquelle

n, $R^3$ et $R^4$ ont les significations indiquées ci-dessus,

avec des isocyanates de formule VIII

$OCN-R^6$ VIII

dans laquelle

$R^6$ a la signification indiquée ci-dessus, ou

b) dans le cas où A représente le radical Ia et $R^4$ représente un atome d'hydrogène, on fait réagir des thiénylisocyanates de formule IX

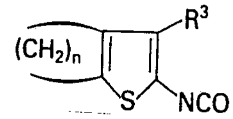

IX

dans laquelle

n et $R^3$ ont les significations indiquées ci-dessus, avec des amines de formule IV

$H-NR^5R^6$ IV

dans laquelle

$R^5$ et $R^6$ ont les significations indiquées ci-dessus, ou

c) dans le cas où A représente le radical Ib, on fait réagir des thiénylamines de formule VII

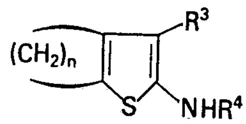

VII

dans laquelle

n, $R^3$ et $R^4$ ont les significations indiquées ci-dessus,

avec des halogénures d'esters d'acides imidocarboniques de formule X

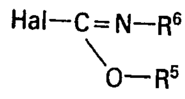

X

dans laquelle

$R^5$ et $R^6$ ont les significations indiquées ci-dessus, et

Hal représente un atome d'halogène.

6. Agents en vue de favoriser le rendement des animaux, caractérisés en ce qu'ils contiennent des thiénylurées ou des thiénylisourées de formule I selon la revendication 1.

7. Aliments et eau de boisson pour animaux, additifs pour aliments et eau de boisson pour les animaux, caractérisés en ce qu'ils contiennent des thiénylurées ou des thiénylisourées de formule I selon la revendication 1.

8. Utilisation de thiénylurées ou de thiénylisourées de formule I selon la revendication 1, en vue de favoriser le rendement des animaux.

9. Procédé de préparation d'agents en vue de favoriser le rendement des animaux, caractérisé en ce qu'on mélange des thiénylurées ou des thiénylisourées de formule I selon la revendication 1 avec des agents délayants et/ou des diluants.

10. Procédé de préparation d'aliments pour animaux, d'eau de boisson pour animaux ou d'additifs pour les aliments et l'eau de boisson pour animaux, caractérisé en ce qu'on mélange des thiénylurées ou des thiénylisourées de formule I selon la revendication 1 avec des aliments ou de l'eau de boisson et éventuellement d'autres substances auxiliaires.